# EUROPEAN PATENT APPLICATION

(11) **EP 4 249 462 A1**
(43) Date of publication of application: **27.09.2023**
(21) Application number: 21894338.9
(22) Date of filing: 01.10.2021
(51) Int. Cl.: C07C 45/61, C07D 333/22, C07C 49/784, C07C 49/84, C07D 295/192, C07C 67/28, C07C 69/157

(54) **METHOD FOR PRODUCING KETONE DERIVATIVE**

(30) Priority: 18.11.2020 JP 2020192037; 11.06.2021 JP 2021098285
(71) Applicant: Tokuyama Corporation, Shunan-shi, Yamaguchi 745-8648 (JP); OSAKA UNIVERSITY, Suita-shi Osaka 565-0871 (JP)
(72) Inventor: SEKI Masahiko, Shunan-shi, Yamaguchi 745-8648 (JP); MASHIMA Kazushi, Suita-shi, Osaka 565-0871 (JP); TSURUGI Hayato, Suita-shi, Osaka 565-0871 (JP); KATO Daiki, Suita-shi, Osaka 565-0871 (JP); MURASE Tomoya, Suita-shi, Osaka 565-0871 (JP); TALODE Jalindar Bhausaheb, Suita-shi, Osaka 565-0871 (JP)
(74) Representative: Hoffmann Eitle
(86) International application number: PCT/JP2021/036517
(87) International publication number: WO 2022/107463

(57) **Abstract**

An object of the present invention is to provide a novel method for producing a ketone derivative, and more specifically, a method for producing a ketone derivative (I) represented by formula (I), including mixing a thioester derivative (II) represented by formula (II), a Grignard reagent (III) represented by formula (III) and a copper salt to form a ketone derivative (I).

## Description

### FIELD OF THE INVENTION

The present invention relates to a method for producing a ketone derivative.

### BACKGROUND ART

SGLT2 inhibitors are useful as an antidiabetic drug. Note that, "SGLT2" refers to sodium-glucose cotransporter-2. Examples of the SGLT2 inhibitors known well include canagliflozin(1-(β-D-glycopyranosyl)-4-methyl-3-[5-(4-fluorophenyl)-2-thienylmethyl]benzene), empagliflozin((1S)-1,5-anhydro-1-C-{4-chloro-3-[(4-{[(3S)-oxolan-3-yl]oxy}phenyl)methyl]phenyl}-D-glucitol), ipragliflozin((1S)-1,5-anhydro-1-C-{3-[(1-benzothiophen-2-yl)methyl]-4-fluorophenyl}-D-glucitol-(2S)-pyrrolidine-2-carboxylic acid), and dapagliflozin((2S,3R,4R,5S,6R)-2-[4-chloro-3-(4-ethyloxybenzyl)phenyl]-6-(hydroxymethyl)tetrahydro-2H-pirane-3,4,5-thiol).

As a method for producing an SGLT2 inhibitor, for example, it has been proposed that canagliflozin is synthesized by deprotecting a protecting group of a 1-(β-D-glycopyranosyl)-4-methyl-3-[5-(4-fluorophenyl)-2-thienylmethyl]benzene precursor (see, Patent Document 1). The precursor (1-(β-D-glycopyranosyl)-4-methyl-3-[5-(4-fluorophenyl)-2-thienylmethyl]benzene precursor) is also called as a C-aryl hydroxy glycoside derivative and attracted attention as an intermediate for producing an SGLT-2 inhibitor (Patent Documents 1 and 2 and Non Patent Documents 1 to 3).

A wide variety of methods for producing a C-aryl hydroxy glycoside derivative have been proposed. Examples of the methods include a method of adding an aryl group to D-gluconolactone derivative by acting aryl lithium on the derivative at an ultra-low temperature of -78°C (Non Patent Documents 1 and 3); a method of adding an aryl group to a D-gluconolactone derivative by acting a Turbo-Grignard reagent such as ArMgBr·LiCl (Ar represents an aryl group) on the derivative at a low temperature of -20 to -10°C (Non Patent Document 2); and a method of adding an aryl group to a D-gluconolactone derivative by using a magnesium ate complex obtained from lithium tri-n-butyl magnesate (nBu₃MgLi) in an environment of about -15°C (Patent Document 2). In the meantime, it has been reported that a ketone derivative is obtained by allowing an organic zinc reagent to react with a thioester derivative in the presence of a nickel catalyst to cause coupling (Non Patent Documents 4 and 5).

Remdesivir represented by the following formula (VI') is a compound used as an antiviral drug. Remdesivir exhibits antiviral activity against, for example, a single stranded RNA virus such as an RS virus and a coronavirus.

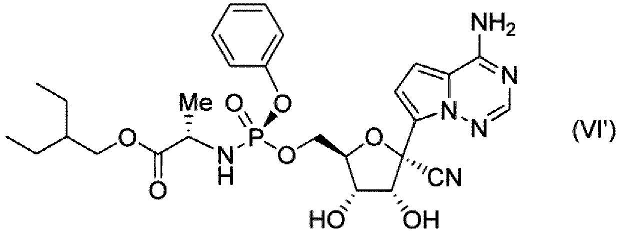

Patent Document 3 discloses a method for producing remdesivir and an intermediate thereof. Patent Document 3 discloses that lactone represented by the following formula (I') is reacted with bromopyrazole represented by the following formula (Ar") in the presence of chlorotrimethylsilane (TMSCI) and n-butyllithium at -78°C to obtain a hydroxy nucleoside represented by the following formula (V'). The hydroxy nucleoside can be used as an intermediate for synthesizing remdesivir.

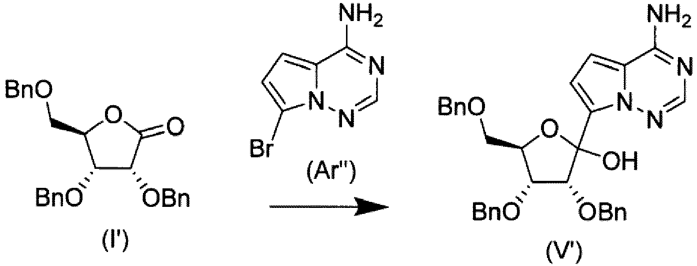

### CITATION LIST

### PATENT DOCUMENTS

Patent Document 1: WO 2010/043682
Patent Document 2: WO 2015/012110
Patent Document 3: WO 2012/012776

### NON PATENT DOCUMENTS

Non Patent Document 1: J. Med. Chem. 2008, 51, 1145-1149
Non Patent Document 2: Org. Lett. 2014, 16, 4090-4093
Non Patent Document 3: J. Org. Chem. 1989, 54, 610-612
Non Patent Document 4: Tetrahedron Letters 2002, 43, 1039-1042
Non Patent Document 5: Chem. Eur. J. 2018, 24, 8774-8778

### SUMMARY OF THE INVENTION

Processes for producing a C-aryl hydroxy glycoside derivative, or remdesivir or an intermediate thereof that have been used heretofore must be all performed by use of expensive reagents in an extremely-low temperature condition. Since equipment cost or running cost result in extremely expensive, it is difficult to produce a final drug substance inexpensively in a large scale. Because of this, it has been desired to develop a ketone derivative production method, which enable industrial production of a C-aryl hydroxy glycoside derivative, or remdesivir or an intermediate thereof, inexpensively and efficiently.

An object of the present invention is to provide a novel method for producing a ketone derivative.

[1] A method for producing a ketone derivative (I) represented by the following formula (I): 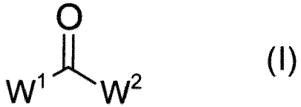 wherein W¹ and W² each independently represent an alkyl group that may have a substituent, an alkenyl group that may have a substituent, a cycloalkyl group that may have a substituent, a heterocycloalkyl group that may have a substituent, an aryl group that may have a substituent, a heteroaryl group that may have a substituent, an arylalkyl group that may have a substituent or an arylalkenyl group that may have a substituent,
   the method comprising a step of mixing:
   a thioester derivative (II) represented by the following formula (II): 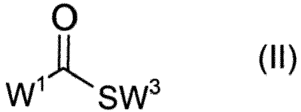
   wherein W¹ is the same as defined above, and W³ represents an alkyl group that may have a substituent, an alkenyl group that may have a substituent, a cycloalkyl group that may have a substituent, a heterocycloalkyl group that may have a substituent, an aryl group that may have a substituent, a heteroaryl group that may have a substituent, an arylalkyl group that may have a substituent or an arylalkenyl group that may have a substituent;
   a Grignard reagent (III) selected from the group consisting of
      a Grignard reagent (IIIa) represented by the following formula (IIIa):

         W²MgX (Ilia)
      wherein W² is the same as defined above, and X represents a halogen atom, and
      a Grignard reagent (IIIb) represented by the following formula (IIIb):

         W²MgX·LiCl (IIIb)
      wherein W² and X are the same as defined above; and
   a copper salt, to form the ketone derivative (I).
[2] The method according to [1], wherein, in the step, the Grignard reagent (III) and the copper salt are mixed to form an organic copper reagent, and thereafter the thioester derivative (II) is mixed to contact the organic copper reagent and the thioester derivative (II) with each other.
[3] The method according to [1] or [2], wherein an amount of the copper salt used is 0.1 mol or more and 1 mol or less relative to 1 mol of the Grignard reagent (III).
[4] The method according to any one of [1] to [3], wherein the thioester derivative (II), the Grignard reagent (III) and the copper salt are mixed in a temperature range of 20°C or more and 60°C or less.
[5] The method according to any one of [1] to [4],
   wherein W² is an aryl group in which a carbon atom adjacent to each of two sides of a carbon atom having a bond of the aryl group has no substituent and the remaining carbon atoms may have a substituent; or a heteroaryl group in which a carbon atom or heteroatom adjacent to each of two sides of a carbon atom having a bond of the heteroaryl group has no substituent and the remaining carbon atoms or heteroatom(s) may have a substituent,
   wherein, in the step, the thioester derivative (II), the Grignard reagent (III), the copper salt and a Grignard reagent (IV) represented by the following formula (IV):

      W⁴MgX¹ (IV)

      wherein W⁴ represents a phenyl group that has a substituent(s) at at least one of ortho positions and that may have a substituent(s) at a meta position(s) and/or a para position, and X¹ represents a halogen atom,
      are mixed.
[6] The method according to [5], wherein an amount of the Grignard reagent (IV) used is 0.01 mol or more and 1 mol or less relative to 1 mol of the Grignard reagent (III).
[7] The method according to [5] or [6], wherein, in the step, the Grignard reagent (III) and the copper salt are mixed, and thereafter the Grignard reagent (IV) is mixed to form an organic copper reagent, and thereafter the thioester derivative (II) is mixed to contact the organic copper reagent and the thioester derivative (II) with each other.

According to the present invention, there are provided a novel method for producing a ketone derivative. According to the production method of the present invention, a ketone derivative can be industrially produced inexpensively and efficiently, and equipment cost, running cost and the like can be significantly suppressed.

### BRIEF DESCRIPTION OF THE DRAWINGS

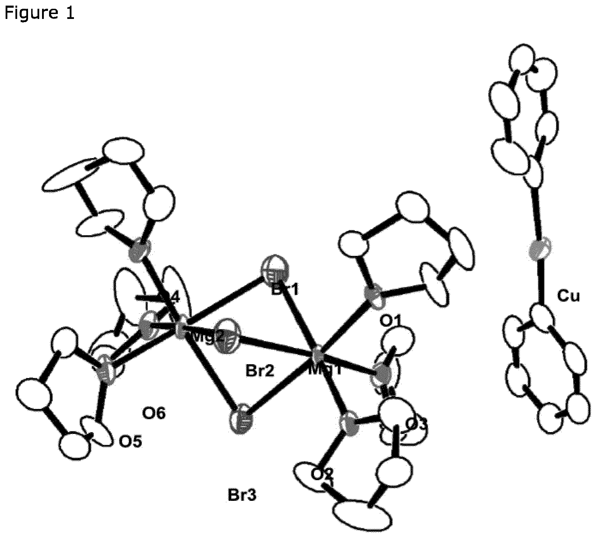

FIG. 1 shows the molecular structure of [Ph₂Cu][Mg₂Br₃(thf)₆] shown by 50% thermal ellipsoid.

### DETAILED DESCRIPTION OF THE INVENTION

Hereinafter, the present invention will be described.

### <Descriptions of Terms>

Hereinafter, the terms used herein will be described. The following descriptions are applied throughout the present specification unless otherwise specified.

### Halogen atom

A halogen atom is selected from a fluorine atom, a chlorine atom, a bromine atom and an iodine atom.

### Alkyl group

The number of carbon atoms of a linear alkyl group is usually 1 to 20, and preferably 1 to 10. The number of carbon atoms of the linear alkyl group is, for example, 1 to 8, 1 to 6, 1 to 5, 1 to 4, 1 to 3 or 1 to 2. The number of carbon atoms of a branched alkyl group is usually 3 to 20, and preferably 3 to 10. The number of carbon atoms of the branched alkyl group is, for example, 3 to 8, 3 to 6, 3 to 5 or 3 to 4.

### Alkenyl group

The number of carbon atoms of a linear alkenyl group is usually 1 to 20, and preferably 1 to 10. The number of carbon atoms of the linear alkenyl group is, for example, 1 to 8, 1 to 6, 1 to 5, 1 to 4, 1 to 3 or 1 to 2. The number of carbon atoms of a branched alkenyl group is usually 3 to 20, and preferably 3 to 10. The number of carbon atoms of the branched alkenyl group is, for example, 3 to 8, 3 to 6, 3 to 5 or 3 to 4.

### Cycloalkyl group

The number of carbon atoms of a cycloalkyl group is usually 3 to 10, preferably 3 to 8, and more preferably 3 to 6.

### Heterocycloalkyl group

A heterocycloalkyl group contains, for examples, one or two heteroatoms independently selected from the group consisting of an oxygen atom, a sulfur atom and a nitrogen atom. The heterocycloalkyl group is, for examples, a 4- to 7-membered heterocycloalkyl group. The heterocycloalkyl group preferably contains an oxygen atom as a heteroatom. Examples of the heterocycloalkyl group include a tetrahydrofuranyl group and a tetrahydropyranyl group. The heterocycloalkyl group is preferably a tetrahydrofuranyl group.

### Aryl group

An aryl group is, for examples, a monocyclic, bicyclic or tricyclic aromatic hydrocarbon ring group having 4 to 14, and preferably 6 to 14 carbon atoms. Examples of the aryl group include a phenyl group and a naphthyl group. The aryl group is preferably a phenyl group.

### Heteroaryl group

A heteroaryl group contains, for examples, one, two or three heteroatoms independently selected from the group consisting of an oxygen atom, a sulfur atom and a nitrogen atom. The heteroaryl group is, for examples, a monocyclic or bicyclic, 4- to 10-membered (preferably 5-to 10-membered) aromatic heterocyclic group. The heteroaryl group is preferably a thienyl group, a benzothiophenyl group, a furyl group, a pyrrolyl group, an imidazolyl group or a pyridyl group, and more preferably a thienyl group or a benzothiophenyl group.

### Haloalkyl group, Haloaryl group and Haloheteroaryl group

A haloalkyl group, a haloaryl group and a haloheteroaryl group are an alkyl group having one or more halogen atoms, an aryl group having one or more halogen atoms and a heteroaryl group having one or more halogen atoms, respectively, and the alkyl group, the aryl group and the heteroaryl group are the same as defined above. The number of halogen atoms of the haloalkyl group, the haloaryl group or the haloheteroaryl group is usually 1 to 3, preferably 1 or 2, and more preferably 1.

### Alkylene group, Arylene group and Heteroarylene group

An alkylene group, an arylene group and a heteroarylene group are divalent functional groups formed by removing one hydrogen atom from an alkyl group, an aryl group and a heteroaryl group, respectively, and the alkyl group, the aryl group and the heteroaryl group are the same as defined above.

### Haloalkylene group, Haloarylene group and Haloheteroarylene group

A haloalkylene group, a haloarylene group and a haloheteroarylene group are divalent functional groups formed by removing one hydrogen atom from a haloalkyl group, a haloaryl group and a haloheteroaryl group, respectively, and the haloalkyl group, the haloaryl group and the haloheteroaryl group are the same as defined above.

### Arylalkyl group

An arylalkyl group is an alkyl group having one or more aryl groups, and the alkyl group and the aryl group are the same as defined above. The number of aryl groups of the arylalkyl group is usually 1 to 3, preferably 1 or 2, and more preferably 1.

### Arylalkenyl group

An arylalkenyl group is an alkenyl group having one or more aryl groups, and the alkenyl group and the aryl group are the same as defined above. The number of aryl groups of the arylalkenyl group is usually 1 to 3, preferably 1 or 2, and more preferably 1.

### Alkylcarbonyl group and Arylcarbonyl group

An alkylcarbonyl group and an arylcarbonyl group are groups represented by formula: -CO-alkyl group and formula: -CO-aryl group, respectively, and the alkyl group and the aryl group are the same as defined above.

### Alkyloxy group, Haloalkyloxy group, Heterocycloalkyloxy group and Arylalkyloxy group

An alkyloxy group, a haloalkyloxy group, a heterocycloalkyloxy group and an arylalkyloxy group are groups represented by formula: -O-alkyl group, formula: -O-haloalkyl group, formula: -O-heterocycloalkyl group and formula: -O-arylalkyl group, respectively, and the alkyl group, the haloalkyl group, the heterocycloalkyl group and the arylalkyl group are the same as defined above.

### Alkylthio group, Haloalkylthio group, Heterocycloalkylthio group and Arylalkylthio group

An alkylthio group, a haloalkylthio group, a heterocycloalkylthio group and an arylalkylthio group are groups represented by formula: -S-alkyl group, formula: -S-haloalkyl group, formula: -S-heterocycloalkyl group and formula: -S-arylalkyl group, respectively, and the alkyl group, the haloalkyl group, the heterocycloalkyl group and the arylalkyl group are the same as defined above.

### Alkyloxycarbonyl group

An alkyloxycarbonyl group is a group represented by formula: -COO-alkyl group, and the alkyl group is the same as defined above. The number of carbon atoms of the alkyl group contained in the alkyloxycarbonyl is preferably 1 to 10, more preferably 1 to 8, still more preferably 1 to 6, and still more preferably 1 to 4.

### Monoalkylamino group

A monoalkylamino group is a group represented by formula: -NH(-Q¹), wherein Q¹ represents an alkyl group, and the alkyl group is the same as defined above. The number of carbon atoms of the alkyl group represented by Q¹ is preferably 1 to 6, more preferably 1 to 4, still more preferably 1 to 3, and still more preferably 1 or 2.

### Dialkylamino group

A dialkylamino group is a group represented by formula: -N(-Q²)(-Q³), wherein Q² and Q³ each independently represent an alkyl group, and the alkyl group is the same as defined above. The number of carbon atoms of the alkyl group represented by Q² or Q³ is preferably 1 to 6, more preferably 1 to 4, still more preferably 1 to 3, and still more preferably 1 or 2.

### Alicyclic amino group

An alicyclic amino group is, for example, a 5- or 6- membered alicyclic amino group. Examples of the 5- or 6-membered alicyclic amino group include a morpholino group, a thiomorpholino group, a pyrrolidin-1-yl group, a pyrazolidin-1-yl group, an imidazolidin-1-yl group and a piperidin-1-yl group. The alicyclic amino group may contain, in addition of a nitrogen atom having a bond of the alicyclic amino group, a heteroatom (for example, one heteroatom) independently selected from the group consisting of an oxygen atom, a sulfur atom and a nitrogen atom. The alicyclic amino group is preferably a morpholino group.

### Aminocarbonyl group, Monoalkylaminocarbonyl group, Dialkylaminocarbonyl group and Alicyclic aminocarbonyl group

An aminocarbonyl group, a monoalkylaminocarbonyl group, a dialkylaminocarbonyl group and an alicyclic aminocarbonyl group are groups represented by formula: -CO-amino group, formula: -CO-monoalkylamino group, formula: -CO-dialkylamino group and formula: - CO-alicyclic amino group, respectively, and the monoalkylamino group, the dialkylamino group and the alicyclic amino group are the same as defined above.

### < Ketone derivative (I) >

A ketone derivative (I) is a compound represented by the following formula (I):

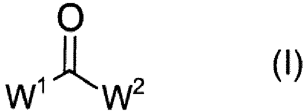

In formula (I), W¹ and W² each independently represent:
(1) an alkyl group that may have a substituent;
(2) an alkenyl group that may have a substituent;
(3) a cycloalkyl group that may have a substituent;
(4) a heterocycloalkyl group that may have a substituent;
(5) an aryl group that may have a substituent;
(6) a heteroaryl group that may have a substituent;
(7) an arylalkyl group that may have a substituent; or
(8) an arylalkenyl group that may have a substituent.

Hereinafter, the functional groups (1) to (8) will be described.

### Alkyl group that may have a substituent

The alkyl group is the same as defined above. The alkyl group may have one or more substituents. The number of substituents is preferably 1 to 3, and more preferably 1 or 2. The one or more substituents can be each independently selected from substituent groups α and β. The one or more substituents may be selected from not only substituent group α but also substituent group β.

### Alkenyl group that may have a substituent

The alkenyl group is the same as defined above. The alkenyl group may have one or more substituents. The number of substituents is preferably 1 to 3, and more preferably 1 or 2. The one or more substituents can be each independently selected from substituent groups α and β. The one or more substituents may be selected from not only substituent group α but also substituent group β.

### Cycloalkyl group that may have a substituent

The cycloalkyl group is the same as defined above. The cycloalkyl group may have one or more substituents. The number of substituents is preferably 1 to 3, and more preferably 1 or 2. The one or more substituents can be each independently selected from substituent groups α and β. The one or more substituents may be selected from not only substituent group α but also substituent group β.

### Heterocycloalkyl group that may have a substituent

The heterocycloalkyl group is the same as defined above. The heterocycloalkyl group may have one or more substituents. The number of substituents is preferably 1 to 3, and more preferably 1 or 2. The one or more substituents can be each independently selected from substituent groups α and β. The one or more substituents may be selected from not only substituent group α but also substituent group β.

### Aryl group that may have a substituent

The aryl group is the same as defined above. The aryl group may have one or more substituents. The number of substituents is preferably 1 to 3, and more preferably 1 or 2. The one or more substituents can be each independently selected from substituent groups α and β. The one or more substituents may be selected from not only substituent group α but also substituent group β.

In the aryl group, a carbon atom adjacent to each of two sides of a carbon atom having a bond of the aryl group (a carbon atom binding to W¹-CO- or W²-CO- in formula (I); a carbon atom binding to Mg in formula (IIIa) or (IIIb)) preferably has no substituent. The remaining carbon atoms may have a substituent.

### Heteroaryl group that may have a substituent

The heteroaryl group is the same as defined above. The heteroaryl group may have one or more substituents. The number of substituents is preferably 1 to 3, and more preferably 1 or 2. The one or more substituents can be each independently selected from substituent groups α and β. The one or more substituents may be selected from not only substituent group α but also substituent group β.

In the heteroaryl group, a carbon atom or heteroatom adjacent to each of two sides of a carbon atom having a bond of the heteroaryl group (a carbon atom binding to W¹-CO- or W²-CO- in formula (I); a carbon atom binding to Mg in formula (IIIa) or (IIIb)) preferably has no substituent. The remaining carbon atoms or heteroatom(s) may have a substituent.

### Arvlalkvl group that may have a substituent

The arylalkyl group is the same as defined above. The arylalkyl group may have one or more substituents. The number of substituents is preferably 1 to 3, and more preferably 1 or 2. The one or more substituents can be each independently selected from substituent groups α and β. The one or more substituents may be selected from not only substituent group α but also substituent group β.

### Arylalkenyl group that may have a substituent

The arylalkenyl group is the same as defined above. The arylalkenyl group may have one or more substituents. The number of substituents is preferably 1 to 3, and more preferably 1 or 2. The one or more substituents can be each independently selected from substituent groups α and β. The one or more substituents may be selected from not only substituent group α but also substituent group β.

### Substituent group α

The substituent group α is composed of the following substituents.
(α-1) Halogen atom
(a-2) Nitrile group
(a-3) Nitro group
(a-4) Amino group
(a-5) Alkyl group
(a-6) Haloalkoxy group
(a-7) Monoalkylamino group
(a-8) Dialkylamino group
(a-9) Alicyclic amino group
(α-10) Alkyloxycarbonyl group
(α-11) Aminocarbonyl group
(α-12) Monoalkylaminocarbonyl group
(α-13) Dialkylaminocarbonyl group
(α-14) Alicyclic aminocarbonyl group
(α-15) Hydroxy group that may be protected with a protecting group
(α-16) Thiol group that may be protected with a protecting group

### Substituent group β

The substituent group β is composed of the following substituents.
(β-1) Substituent represented by formula (i)
(β-2) Substituent represented by formula (ii)

Hereinafter, the substituent group α and β will be described.

In (a-5) and (a-6), the number of carbon atoms of the alkyl group is preferably 1 to 10, more preferably 1 to 8, still more preferably 1 to 6, still more preferably 1 to 4, still more preferably 1 to 3, still more preferably 1 to 2. In (a-6), the number of halogen atoms that the alkyl group has is preferably 1 to 3, more preferably 1 to 2, and still more preferably 1.

### Hydroxy group that may be protected with a protecting group

A hydroxy group protecting group is preferably a group that can protect a hydroxy group during an intended reaction and be eliminated from the hydroxy group after completion of the intended reaction. Examples of the hydroxy group protecting group include an alkylcarbonyl-type protecting group, an arylcarbonyl-type protecting group, an arylalkyl-type protecting group, an alkyl-type protecting group, an arylalkyloxyalkyl-type protecting group, an alkyloxyalkyl-type protecting group, a silyl-type protecting group, an oxycarbonyl-type protecting group, an acetal-type protecting group and an aryl-type protecting group. These protecting groups may have one or more halogen atoms.

Examples of the alkylcarbonyl-type protecting group include a C₁₋₁₀ alkylcarbonyl group that may have one or more substituents. The substituent can be selected from, for example, a halogen atom, a nitro group, a cyano group, a phenyl group; a C₁₋₁₀ alkyl group, preferably a C₁₋₈ alkyl group, more preferably a C₁₋₆ alkyl group, and still more preferably a C₁₋₄ alkyl group; a C₁₋₁₀ alkyloxy group, preferably a C₁₋₈ alkyloxy group, more preferably a C₁₋₆ alkyloxy group, and still more preferably a C₁₋₄ alkyloxy group; and a C₂₋₁₁ alkyloxycarbonyl group, preferably a C₂₋₉ alkyloxycarbonyl group, more preferably a C₂₋₇ alkyloxycarbonyl group, and still more preferably a C₂₋₅ alkyloxycarbonyl group. Examples of the C₁₋₁₀ alkylcarbonyl group that may have one or more substituents include an acetyl group, a propanoyl group, a butanoyl group, an isopropanoyl group and a pivaloyl group. The alkylcarbonyl-type protecting group is preferably a C₁₋₅ alkylcarbonyl group, more preferably an acetyl group or pivaloyl group, and still more preferably an acetyl group.

Examples of the arylcarbonyl-type protecting group include a C₆₋₁₀ arylcarbonyl group that may have one or more substituents. Examples of the substituents are the same as those of alkylcarbonyl-type protecting group. Examples of the C₆₋₁₀ arylcarbonyl group that may have one or more substituents include a benzoyl group, a 4-nitrobenzoyl group, a 4-methyloxybenzoyl group, a 4-methylbenzoyl group, a 4-tert-butylbenzoyl group, a 4-fluorobenzoyl group, a 4-chlorobenzoyl group, a 4-bromobenzoyl group, a 4-phenylbenzoyl group and a 4-methyloxycarbonylbenzoyl group.

Examples of the arylalkyl-type protecting group include a C₇₋₁₁ arylalkyl group that may have one or more substituents. Examples of the substituents are the same as those of the alkylcarbonyl-type protecting group. Examples of the C₇₋₁₁ arylalkyl group that may have one or more substituents include a benzyl group, a 1-phenylethyl group, a diphenylmethyl group, a 1,1-diphenylethyl group, a naphthylmethyl group and a trityl group. The arylalky-type protecting group is preferably a benzyl group.

Examples of the alkyl-type protecting group include a C₁₋₁₀ alkyl group that may have one or more substituents. Examples of the substituents are the same as those of the alkylcarbonyl-type protecting group. The alkyl-type protecting group is preferably a C₁₋₅ alkyl group that may have one or more substituents, more preferably a methyl group, an ethyl group and a tert-butyl group, and still more preferably a methyl group.

Examples of the arylalkyloxyalkyl-type protecting group include arylalkyloxyalkyl groups such as a C₇₋₁₁ arylalkyloxymethyl group that may have one or more substituents, a C₇₋₁₁ arylalkyloxyethyl group that may have one or more substituents and a C₇₋₁₁ arylalkyloxypropyl group that may have one or more substituents. Examples of the substituents are the same as those of the alkylcarbonyl-type protecting group. Examples of the arylalkyloxyalkyl-type protecting group include a benzyloxymethyl group that may have one or more substituents, preferably a benzyloxymethyl group that may be substituted with a halogen atom, a nitro group, a cyano group, a methyl group or a methyloxy group, and more preferably a benzyloxymethyl group.

Examples of the alkyloxyalkyl-type protecting group include alkyloxyalkyl groups such as a C₁₋₁₀ alkyloxymethyl group that may have one or more substituents, a C₁₋₁₀ alkyloxyethyl group that may have one or more substituents and a C₁₋₁₀ alkyloxypropyl group that may have one or more substituents. Examples of the substituents are the same as those of the alkylcarbonyl-type protecting group. The alkyloxyalkyl-type protecting group is preferably a C₁₋₁₀ alkyloxymethyl group that may have one or more substituents, more preferably a C₁₋₅ alkyloxymethyl group having a halogen atom, a nitro group, a cyano group, a methyloxy group or an ethyloxy group, and still more preferably a methyloxymethyl group.

Examples of the silyl-type protecting group include a silyl group having a functional group selected from a C₁₋₁₀ alkyl group that may have one or more substituents, a C₇₋₁₀ arylalkyl group that may have one or more substituents and a C₆₋₁₀ aryl group that may have one or more substituents. Examples of the substituents are the same as those of the alkylcarbonyl-type protecting group. The silyl-type protecting group is preferably a silyl group having a functional group selected from a C₁₋₁₀ alkyl group and a C₆₋₁₀ aryl group, more preferably a silyl group having a functional group selected from a C₁₋₅ alkyl group and a phenyl group, and still more preferably a trimethylsilyl group, a triethylsilyl group, a tert-butyldimethylsilyl group or a tert-butyldiphenylsilyl group.

Examples of the oxycarbonyl-type protecting group include a C₁₋₁₀ alkyloxycarbonyl group that may have one or more substituents, a C₂₋₁₀ alkenyloxycarbonyl group that may have one or more substituents, and a C₇₋₁₁ arylalkyloxycarbonyl group that may have one or more substituents. Examples of the substituents are the same as those of the alkylcarbonyl-type protecting group. The oxycarbonyl-type protecting group is preferably a C₁₋₅ alkyloxycarbonyl group, a C₂₋₅ alkenyloxycarbonyl group or benzyloxycarbonyl group, and more preferably a methyloxymethyl group, an allyloxycarbonyl group or a benzyloxycarbonyl group.

Examples of the acetal-type protecting group include a tetrahydrofuranyl group and a tetrahydropyranyl group.

Examples of the aryl-type protecting group include aryl groups such as a phenyl group.

A hydroxy group protected with a protecting group is preferably a group represented by formula: -O-R. R represents an alkyl group, a haloalkyl group, an aryl group, a haloaryl group, a heterocycloalkyl group, an alkylcarbonyl group, an arylcarbonyl group or an arylalkyl group. The number of carbon atoms of a group represented by formula: -O-R is preferably 1 to 10, and more preferably 1 to 8. R is preferably an alkyl group, a heterocycloalkyl group, an alkylcarbonyl group or an arylalkyl group, and more preferably an ethyl group, a tetrahydrofuranyl group, an acetyl group or a benzyl group.

### Thiol group that may be protected with a protecting group

A thiol group protecting group is preferably a group that can protect the thiol group during an intended reaction and be eliminated from the thiol group after completion of the reaction. Examples of the thiol group protecting group include an alkylcarbonyl-type protecting group, an arylcarbonyl-type protecting group, an arylalkyl-type protecting group, an alkyl-type protecting group, an arylalkyloxyalkyl-type protecting group, an alkyloxyalkyl-type protecting group, a silyl-type protecting group, an oxycarbonyl-type protecting group, an acetal-type protecting group, and an aryl-type protecting group. These protecting groups may have one or more halogen atoms. These protecting groups are the same as defined above.

A thiol group protected with a protecting group is preferably a group represented by formula: -S-R. R is the same as defined above.

### Substituent represented by formula (i)

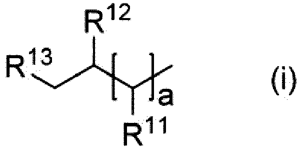

In formula (i), R¹¹, R¹² and R¹³ each independently represent an alkyl group, a haloalkyl group, an aryl group, a haloaryl group or a hydroxy group that may be protected with a protecting group. The hydroxy group that may be protected with a protecting group is preferably a group represented by the above formula: -O-R. "a" represents 0 or more and 3 or less.

### Substituent represented by formula (ii)

-(V¹⁰)_{b}-(W¹⁰)_{c}-(X¹⁰) (ii)

In formula (ii), V¹⁰ represents an alkylene group, a haloalkylene group, an arylene group, a haloarylene group, a heteroarylene group, a haloheteroarylene group, an ester bond, an ether bond or a carbonyl group. The number of carbon atoms of the alkylene group or haloalkylene group is preferably 1 to 10, and more preferably 1 to 8. The number of carbon atoms of the arylene group, haloarylene group, heteroarylene group or haloheteroarylene group is preferably 4 to 14, and more preferably 6 to 14. V¹⁰ represents preferably an alkylene group, and more preferably a methylene group or an ethylene group.

In formula (ii), "b" represents 0 or 1 and preferably 1.

In formula (ii), W¹⁰ represents an alkylene group, a haloalkylene group, an arylene group, a haloarylene group, a heteroarylene group, a haloheteroarylene group, an ester bond, an ether bond or a carbonyl group. W¹⁰ is preferably a heteroarylene group, more preferably a 5-membered heteroarylene group containing a sulfur atom as a heteroatom.

In formula (ii), "c" represents 0 or 1 and preferably 1.

In formula (ii), X¹⁰ represents a hydrogen atom, an alkyl group that may have a substituent, an aryl group that may have a substituent, or a heteroaryl group that may have a substituent.

The alkyl group, aryl group or heteroaryl group represented by X¹⁰ may have one or more substituents. The one or more substituents can be each independently selected from the substituent group a. The one or more substituents each independently selected preferably from a halogen atom, an alkyl group, a haloalkyl group, an alkyloxy group, a haloalkyloxy group, an alkylthio group, a haloalkylthio group, a heterocycloalkyloxy group and a heterocycloalkylthio group, more preferably from a halogen atom, a C₁₋₃ alkyloxy group and a heterocycloalkyloxy group, and still more preferably from a fluorine atom, an ethyloxy group and a tetrahydrofuranyloxy group.

X¹⁰ represents preferably an aryl group that may have a substituent or a heteroaryl group that may have a substituent, more preferably an aryl group having a halogen atom, having a C₁₋₃ alkyloxy group or having a heterocycloalkyloxy group containing an oxygen atom as a heteroatom, or an unsubstituted heteroaryl group, and more preferably a phenyl group having a fluorine atom having an ethyloxy group or having a tetrahydrofuranyloxy group, or an unsubstituted benzothiophenyl group.

W¹ is preferably represented by the following formula (iii).

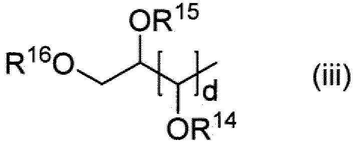

In formula (iii), R¹⁴, R¹⁵ and R¹⁶ each independently represent an alkyl group, a haloalkyl group, an aryl group, a haloaryl group, an alkylcarbonyl group, an arylcarbonyl group or an arylalkyl group. It is preferable that R¹⁴ and R¹⁶ are mutually the same substituent and that R¹⁵ is a different type of substituent from R¹⁴ and R¹⁶. R¹⁴ and R¹⁶ are each preferably an alkylcarbonyl group or an arylalkyl group, preferably an acetyl group or a benzyl group, and more preferably a benzyl group. R¹⁵ is preferably an alkylcarbonyl group or an arylalkyl group, more preferably an acetyl group or a benzyl group, and still more preferably an acetyl group.

In formula (iii), "d" represents 1 or more and 5 or less and is preferably 2 or 3.

W² is preferably represented by the following formula (iv).

-(Y¹⁰)-(V¹⁰)_{b}-(W¹⁰)_{c}-(X¹⁰) (iv)

In formula (iv), Y¹⁰ represents an alkylene group that may have a substituent, an arylene group that may have a substituent or a heteroarylene group that may have a substituent. The number of carbon atoms of the alkylene group is preferably 1 to 10, and more preferably 1 to 8. The number of carbon atoms of the arylene group or heteroarylene group is preferably 4 to 14 and more preferably 6 to 14.

The alkylene group, arylene group and heteroarylene group represented by Y¹⁰ may each have one or more substituents, which can be each independently selected from substituent group a. The one or more substituents are each independently selected preferably from a halogen atom, an alkyl group, a haloalkyl group, an alkyloxy group, a haloalkyloxy group, an alkylthio group and haloalkylthio group, and more preferably from a halogen atom, a C₁₋₃ alkyl group and a C₁₋₃ alkyloxy group.

Y¹⁰ is preferably an arylene group having a substituent; more preferably an arylene group having a halogen atom or a C₁₋₃ alkyl group, and more still preferably a phenylene group having a fluorine atom, a chlorine atom or a methyl group.

Y¹⁰ is preferably an arylene group in which a carbon atom adjacent to each of two sides of a carbon atom binding to W¹-CO- has no substituent and the remaining carbon atoms may have a substituent, or a heteroarylene group in which a carbon atom or heteroatom adjacent to each of two sides of a carbon atom binding to W¹-CO- has no substituent and the remaining carbon atoms or heteroatom(s) may have a substituent. Y¹⁰ is more preferably a phenylene group that has no substituents at the ortho-positions relative to the carbon atom binding to W¹-CO- and that may have a substituent(s) at the meta-position(s) and/or para-position.

In formula (iv), V¹⁰, W¹⁰, X¹⁰, b and c are the same as defined in formula (ii).

Alternatively, W² is preferably represented by the following formula (vi).

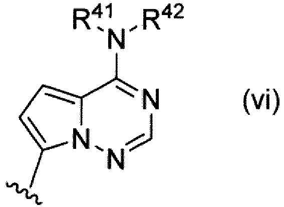

In formula (vi), R⁴¹ and R⁴² each independently represent a hydrogen atom or a protecting group for an amino group. As the protecting group for an amino group, any one of protecting groups including carbamate series, acyl series, amide series, sulfonamide series and phthaloyl groups may be used. Examples of the carbamate-series protecting group include a tert-butoxycarbonyl group, a benzyloxycarbonyl group, a 9-fluorenylmethyloxycarbonyl group, a 2,2,2-trichloroethoxycarbonyl group and an allyloxycarbonyl group. Examples of the acyl-series protecting group include an acetyl group, a pivaloyl group and a benzoyl group. Examples of the amide-series protecting group include a trifluoroacetyl group. Examples of the sulfonamide-series protecting group include a p-toluenesulfonyl group and a 2-nitrobenzenesulfonyl group. Examples of the protecting group for an amino group is preferably an acyl-series or amide-series protecting group and more preferably a pivaloyl group or a trifluoroacetyl group. R⁴¹ and R⁴² may mutually bind to form, e.g., a phthaloyl group serving as protecting group for an amino group. In cases where W² has the structure represented by formula (vi), the compound can be suitably used as an intermediate of remdesivir.

A ketone derivative (I) according to an embodiment is, for example, a compound wherein W¹ and W² each independently represent an aryl group that may have a substituent. Examples of the ketone derivative (I) include the following compounds.

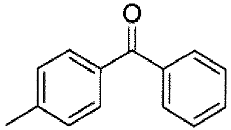

Examples of the compound wherein W¹ and W² each independently represent an aryl group that may have a substituent include the following compounds. Note that, "Me" stands for a methyl group, "Et" stands for an ethyl group, "^{t}Bu" stands for a tert-butyl group, and "Ph" stands for a phenyl group (the same applies throughout the specification).

**[Table 1]**

| | | |
|---|---|---|
| 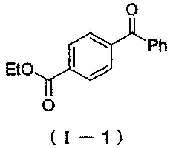 | 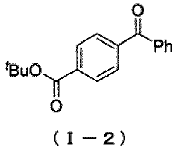 | 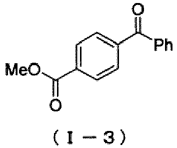 |
| 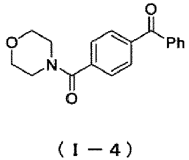 | 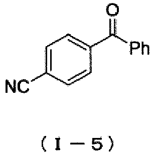 | 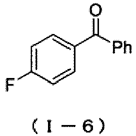 |
| 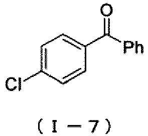 | 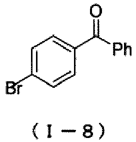 | 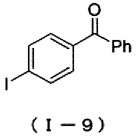 |
| 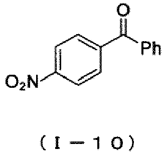 | 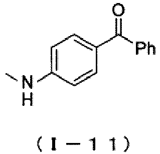 | 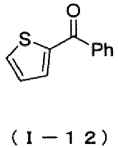 |
| 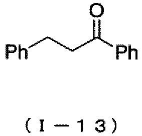 | 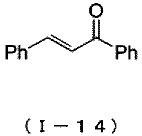 | 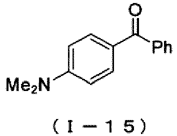 |
| 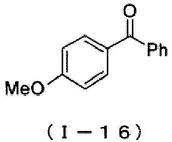 | 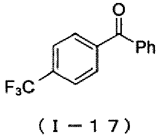 | |

Of compounds I-1 to I-17, compounds I-1 to I-9, I-12 and I-13 are preferable.

A ketone derivative (I) according to another embodiment is, for example, a ketone derivative (Ia) represented by the following formula (Ia).

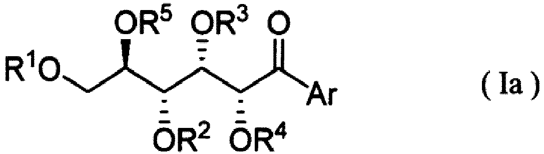

Ar is an alkyl group that may have a substituent, an alkenyl group that may have a substituent, a cycloalkyl group that may have a substituent, a heterocycloalkyl group that may have a substituent, an aryl group that may have a substituent, a heteroaryl group that may have a substituent, an arylalkyl group that may have a substituent or an arylalkenyl group that may have a substituent, and preferably an aryl group that may have a substituent.

It is preferable that R¹ and R² each independently represent a hydroxy group protecting group or a hydrogen atom, R³ and R⁴ each independently represent a hydroxy group protecting group or a hydrogen atom, and R⁵ represents a hydroxy group protecting group; and more preferable that R¹ and R² each independently represent a hydroxy group protecting group, R³ and R⁴ each independently represent a hydroxy group protecting group or a hydrogen atom, and R⁵ represents a hydroxy group protecting group.

R¹ and R² may be hydrogen atoms, and are preferably hydroxy group protecting groups in view of efficient formation of a 6-membered ring compound represented by formula (IVa) later described. R¹ and R² may be the same type of hydroxy group protecting groups or different type of hydroxy group protecting groups, but are preferably the same type of hydroxy group protecting groups in view of efficient introduction and removal of the hydroxy group protecting groups.

R³ and R⁴ may be hydrogen atoms, and are preferably hydroxy group protecting groups in view of efficient formation of a 6-membered ring compound represented by formula (IVa) later described. R³ and R⁴ may be the same type of hydroxy group protecting groups or different hydroxy group protecting groups, but are preferably the same type of hydroxy group protecting groups in view of efficient introduction and removal of the hydroxy group protecting groups. R³ and R⁴ may be the same type of hydroxy group protecting groups as R¹ and R² or different hydroxy group protecting groups from R¹ and R² but are preferably the same type of hydroxy group protecting groups in view of efficient introduction and removal of the hydroxy group protecting groups.

R⁵ is preferably a hydroxy group protecting group different from those represented by R¹ and R². If the hydroxy group protecting group represented by R⁵ is removed, this will allow a hydroxy group to emerge and react with a carbonyl group within the same molecule to form a ring represented by formula (IVa) later described. Accordingly, it is preferable to select the type of hydroxy group protecting group represented by R⁵ such that the hydroxy group protecting group represented by R⁵ can be selectively removed while maintaining hydroxy group protecting groups represented by R¹ and R².

In an embodiment, the hydroxy group protecting group represented by R⁵ is an acetyl group or a pivaloyl group, and the hydroxy group protecting group represented by R¹ and the hydroxy group protecting group represented by R² each independently are a methyl group, a benzyl group, a trimethylsilyl group, a tert-butyldimethylsilyl group or a tert-butyldiphenylsilyl group.

In another embodiment, the hydroxy group protecting group represented by R⁵ is a trimethylsilyl group, a tert-butyldimethylsilyl group or a tert-butyldiphenylsilyl group, and the hydroxy group protecting group represented by R¹ and the hydroxy group protecting group represented by R² each independently are a methyl group, a benzyl group, an acetyl group or a pivaloyl group.

R⁵ is preferably a hydroxy group protecting group different from R¹, R², R³ and R⁴. For example, in cases where R¹, R², R³ and R⁴ are each a benzyl group, R⁵ is preferably a hydroxy group protecting group (for example, an acetyl group) other than a benzyl group. In cases where R⁵ is a hydroxy group protecting group different from R¹, R², R³ and R⁴, -OR⁵ can be eliminated while maintaining -OR¹, -OR², -OR³ and -OR⁴ and a 6-membered ring represented by formula (IVa) later described can be efficiently formed.

In an embodiment, the hydroxy group protecting group represented by R⁵ is an acetyl group or a pivaloyl group, and the hydroxy group protecting groups represented by R¹, R², R³ and R⁴ each independently are a methyl group, a benzyl group, a trimethylsilyl group, a tert-butyldimethylsilyl group or a tert-butyldiphenylsilyl group.

In another embodiment, the hydroxy group protecting group represented by R⁵ is a trimethylsilyl group, a tert-butyldimethylsilyl group or a tert-butyldiphenylsilyl group, and the hydroxy group protecting groups represented by R¹, R², R³ and R⁴ each independently are a methyl group, a benzyl group, an acetyl group or a pivaloyl group.

The ketone derivative (Ia) is a compound wherein, for example, R¹ to R⁵ each independently represent a hydroxy group protecting group and Ar represents an aryl group that may have a substituent. Examples of the compound mentioned above include the following compounds. Note that, "Ac" stands for an acetyl group and "Bn" stands for a benzyl group (the same applies throughout the specification).

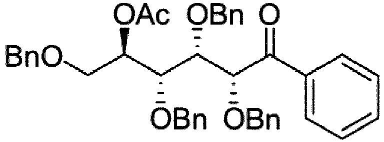

In the above compound, the phenyl group corresponding to Ar may have one or more substituents. The number of substituents is preferably 1 to 3, and more preferably 1 or 2. The one or more substituents can be each independently selected from substituent groups α and β. The one or more substituents may be selected from not only substituent group α but also substituent group β.

Ar is preferably the same as a functional group that an SGLT-2 inhibitor has or as a functional group derived from the functional group that an SGLT-2 inhibitor has in view of usage of a ketone derivative (Ia) as a starting material for producing an SGLT-2 inhibitor or a derivative thereof.

Herein, the SGLT-2 inhibitors including canagliflozin(1-(β-D-glycopyranosyl)-4-methyl-3-[5-(4-fluorophenyl)-2-thienylmethyl]benzene), empagliflozin((1S)-1,5-anhydro-1-C-{4-chloro-3-[(4-{[(3S)-oxolan-3-yl]oxy}phenyl)methyl]phenyl}-D-glucitol), ipragliflozin((1S)-1,5-anhydro-1-C-{3-[(1-benzothiophen-2-yl)methyl]-4-fluorophenyl}-D-glucitol-(2S)-pyrrolidine-2-carboxylic acid) and dapagliflozin((2S,3R,4R,5S,6R)-2-[4-chloro-3-(4-ethyloxybenzyl)phenyl]-6-(hydroxymethyl)tetrahydro-2H-piran-3,4,5-thiol), have a functional group represented by the following formula (A).

Accordingly, Ar is preferably a functional group represented by the following formula (A).

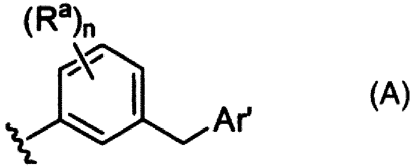

In formula (A), n represents an integer of 0 to 4, preferably 1 to 3, more preferably 1 or 2, and still more preferably 1. In cases where n represents 2 or more, the n number of R^{a} may be the same or different.

In formula (A), the n number of R^{a} can be each independently selected from substituent group a. The n number of R^{a} is each independently selected preferably from a halogen atom, an alkyl group, a haloalkyl group, an alkyloxy group, a haloalkyloxy group, an alkylthio group and a haloalkylthio group, and more preferably form a halogen atom, a C₁₋₃ alkyl group and a C₁₋₃alkyloxy group.

In formula (A), Ar' is a group represented by the following formula (v).

-(W¹⁰)_{c}-(X¹⁰) (v)

In formula (v), W¹⁰, X¹⁰ and c are the same as defined in formula (ii).

In formula (A), Ar' is preferably a group represented by the following formula (Ar'-1), (Ar'-2) or (Ar'-3).

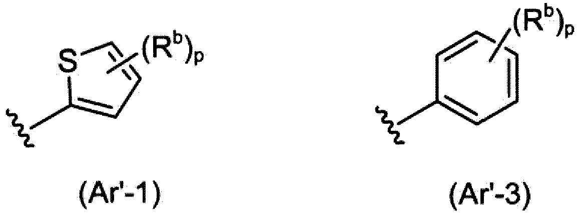

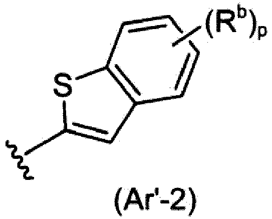

In formulas (Ar'-1), (Ar'-2) and (Ar'-3), p is an integer of 0 to 5. p is preferably an integer of 0 to 3, more preferably an integer of 0 to 2, and still more preferably 0 or 1.

In formulas (Ar'-1), (Ar'-2) and (Ar'-3), the p number of R^{b} can be each independently selected from substituent group a, an aryl group that may have one or more substituents selected from substituent group α and a heteroaryl group that may have one or more substituents selected from substituent group a. The p number of R^{b} are each independently selected from substituent group a, and an aryl group that may have one or more substituents selected from substituent group a. The one or more substituents selected from substituent group α each independently are selected preferably from a halogen atom, an alkyl group, a haloalkyl group, an alkyloxy group, a haloalkyloxy group, an alkylthio group, a haloalkylthio group, a heterocycloalkyloxy group and heterocycloalkylthio group, more preferably from a halogen atom, a C₁₋₃ alkyl group, a C₁₋₃ alkyloxy group and a heterocycloalkyloxy group, and still more preferably from a fluorine atom, an ethyloxy group and a tetrahydrofuranyloxy group.

In cases where p represents 2 or more, the p number of R^{b} may be the same or different.

In formula (Ar'-1), p is preferably 1, and R^{b} is preferably a phenyl group that may have a substituent, more preferably a phenyl group having a halogen atom, and still more preferably a phenyl group having a fluorine atom. The position at which the unsubstituted or substituted phenyl group is bonded is preferably position 2 of the thiophene ring. In the phenyl group having a halogen atom, the position at which the halogen atom is bonded is preferably position 4 of the benzene ring.

In formula (Ar'-2), p is preferably 0.

In formula (Ar'-3), p is preferably 1, and R^{b} is preferably an alkyloxy group that may have a substituent or a heterocycloalkyloxy group that may have a substituent. The alkyloxy group that may have a substituent is preferably a C₁₋₃ alkyloxy group, and more preferably a methoxy group or an ethoxy group. The heterocycloalkyloxy group that may have a substituent is preferably a tetrahydrofuranyloxy group. The position at which the alkyloxy group that may have a substituent or the heterocycloalkyloxy group that may have a substituent is bonded is preferably position 4 of the benzene ring.

In cases where n = 1, Ar is preferably a group represented by the following formula (B).

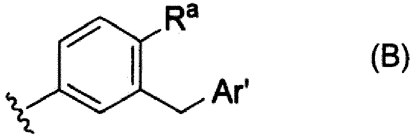

In formula (B), R^{a} and Ar' are the same as defined in formula (A).

Ar is preferably a group represented by the following formula (Ar-1), (Ar-2), (Ar-3) or (Ar-4). Note that, "Et" stands for an ethyl group.

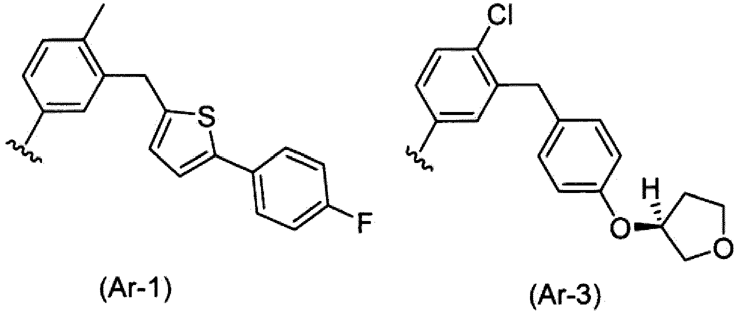

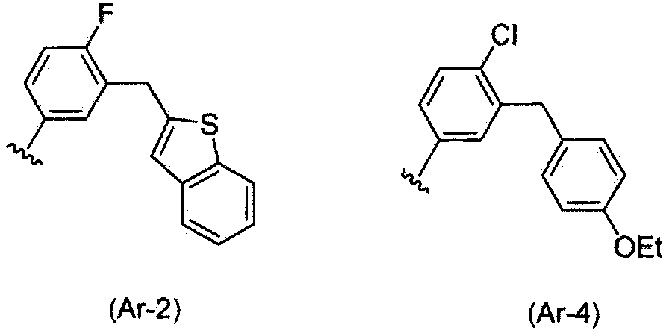

The ketone derivative (Ia) is a compound wherein, for example, R¹ to R⁵ each independently represent a hydroxy group protecting group and Ar represents a group represented by formula (Ar-1). Examples of the compound mentioned above include the following compounds. Note that, "Ac" stands for an acetyl group and "Bn" stands for a benzyl group.

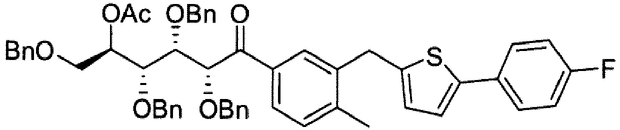

The ketone derivative (I) according to another embodiment is, for example, a ketone derivative (Ib) represented by the following formula (Ib).

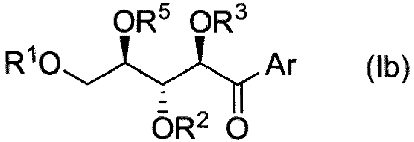

In formula (Ib), R¹, R², R³, R⁵ and Ar each are the same as defined above.

### <Thioester derivative (II)>

A thioester derivative (II) is a compound represented by the following formula (II):

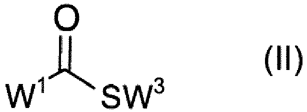

In formula (II), W¹ is the same as defined above and W³ represents:
an alkyl group that may have a substituent;
an alkenyl group that may have a substituent;
a cycloalkyl group that may have a substituent;
a heterocycloalkyl group that may have a substituent;
an aryl group that may have a substituent;
a heteroaryl group that may have a substituent;
an arylalkyl group that may have a substituent; or
an arylalkenyl group that may have a substituent. The alkyl group that may have a substituent; the alkenyl group that may have a substituent; the cycloalkyl group that may have a substituent; the heterocycloalkyl group that may have a substituent; the aryl group that may have a substituent; the heteroaryl group that may have a substituent; the arylalkyl group that may have a substituent; and the arylalkenyl group that may have a substituent are the same as defined above.

W³ may be the same as or different from W¹ and/or W².

The thioester derivative (II) may be commercially available compounds or produced in accordance with a conventional method.

In an embodiment, W¹ is an alkyl group that may have a substituent. In the embodiment, W¹ is preferably an alkyl group that may have a hydroxy group that may be protected by a protecting group and more preferably an alkyl group having a hydroxy group that may be protected by a protecting group. The thioester derivative (II) according to the embodiment is, for example, a thioester derivative (IIa) represented by the following formula (IIa).

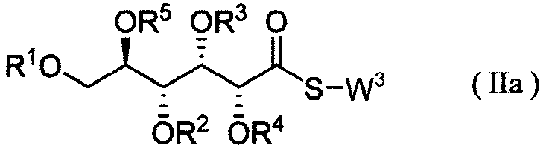

In formula (IIa), R¹ to R⁵ and W³ are the same as defined above.

In an embodiment, W³ is an alkyl group that may have a substituent. In the embodiment, W³ is more preferably an alkyl group having 1 to 20 carbon atoms that may have a substituent, more preferably, an alkyl group having 1 to 16 carbon atoms that may have a substituent and more preferably an alkyl group having 1 to 12 carbon atoms that may have a substituent. Examples of the thioester derivative (IIa) according to the embodiment include the following compounds. Note that, "Ac" stands for an acetyl group and "Bn" stands for a benzyl group.

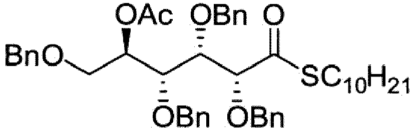

In the above compound, -C₁₀H₂₁ corresponding to W³ may have one or more substituents. The number of substituents is preferably 1 to 3, and more preferably 1 or 2. The one or more substituents can be each independently selected from substituent groups α and β. The one or more substituents may be selected from not only substituent group α but also substituent group β.

In the above compound, -C₁₀H₂₁ corresponding to W³ may be replaced by another alkyl group such as -C₁₂H₂₅. The other alkyl group may have one or more substituents. The number of substituents is preferably 1 to 3, and more preferably 1 or 2. The one or more substituents can be each independently selected from substituent groups α and β. The one or more substituents may be selected from not only substituent group α but also substituent group β.

Examples of the thioester derivative (II) according to another embodiment include a thioester derivative (IIb) represented by the following formula (IIb).

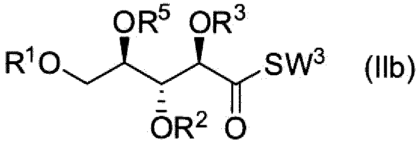

In formula (IIb), R¹, R², R³, R⁵ and W³ are the same as defined above.

### <Method for producing a thioester derivative>

The thioester derivative (II) can be produced, for example, by the following method.

A lactone derivative (V) represented by the following formula (V): 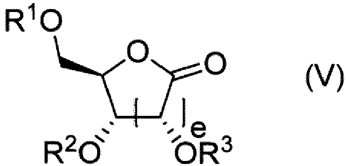 and a thiol (1) represented by the following formula (1):

W³-SH (1)

can be contacted with each other in the presence of a trialkylaluminum to obtain a hydroxyl group-containing compound (II-i) represented by the following formula (II-i):

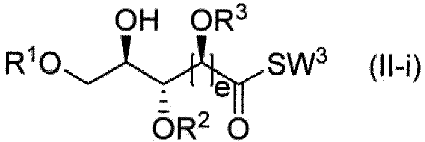

The hydroxyl group of the hydroxyl group-containing compound (II-i) can be protected to obtain a thioester derivative (II-ii) represented by the following formula (II-ii):

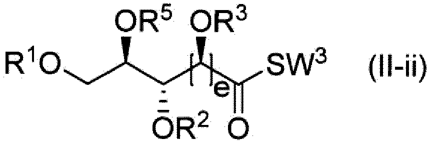

In formula (V), R¹, R² and R³ are the same as defined above; and "e" is 1 or more and 4 or less.

As the lactone derivative (V), a commercially available product or a synthesized product by a known method may be used.

In formula (1), W³ is the same as defined above.

As the thiol (1), a commercially available product or a synthesized product by a known method may be used. At least one selected from the group consisting of 1-decanethiol and 1-dodecanethiol is preferably included as the thiol (1).

In formula (II-i), R¹, R², R³, W³ and e are the same as defined above.

In formula (II-ii), R¹, R², R³, R⁵, W³ and e are the same as defined above.

The amount of the thiol (1) is, for example, 0.5 mol or more and 10 mol or less, preferably 1 mol or more and 5 mol or less, and more preferably 1 mol or more and 2 mol or less, relative to 1 mol of the lactone derivative (V). The amount by mole of the thiol (1) is preferably larger than that of the lactone derivative (V).

A trialkyl aluminum serves as a reactant. The trialkyl aluminum preferably includes at least one of the group consisting of trimethylaluminum, triethylaluminum and tripropylaluminum, and more preferably includes trimethylaluminum.

The amount of the trialkyl aluminum is, for example, 1 mol or more and 10 mol or less, preferably 1 mol or more and 5 mol or less, and more preferably 1 mol or more and 3 mol or less, relative to 1 mol of the lactone derivative (V). The amount by mole of the trialkyl aluminum is preferably larger than that of the lactone derivative (V).

The amount of the trialkyl aluminum is, for example, 1 mol or more and 2 mol or less, preferably 1 mol or more and 1.5 mol or less, and more preferably 1 mol or more and 1.1 mol or less, relative to 1 mol of the thiol (1).

The lactone derivative (V) and the thiol (1) are contacted with each other in the presence of a trialkylaluminum at a contact temperature of for example, -30°C or more and 80°C or less, preferably -10°C or more and 40°C or less, and more preferably -10°C or more and 10°C or less. The contact time is, for example, 30 minutes or more and 120 hours or less, preferably one hour or more and 100 hours or less, and more preferably 30 hours or more and 90 hours or less. The lactone derivative (V) and the thiol (1) are preferably stirred during the contact time while they are kept at the contact temperature mentioned above. The reaction is preferably carried out in an atmosphere of an inert gas such as argon.

The lactone derivative (V) and the thiol (1) are preferably contacted in the presence of a first reaction solvent. In cases where the first reaction solvent is used, the lactone derivative (V) and the thiol (1) are preferably contacted in accordance with the following method. First of all, the lactone derivative (V), the thiol (1) and the trialkylaluminum are separately mixed with the first reaction solvent to prepare a lactone-derivative-containing solution, a thiol-containing solution and a trialkylaluminum-containing solution, respectively. Next, to the thiol-containing solution, the trialkylaluminum-containing solution is added at a rate of, for example, 0.1 mL or more and 10 mL or less per minute and then the mixture is stirred for one minute or more and one hour or less. To the mixed solution after the stirring, the lactone-derivative-containing solution is added at a rate of 0.1 mL or more and 10 mL or less per minute and then the mixture is stirred for 20 minutes or more and 3 hours or less.

In the lactone-derivative-containing solution, the volume of the first reaction solvent per 1 g of lactone derivative (V) is preferably 1 mL or more and 10 mL or less. In the thiol-containing solution, the volume of the first reaction solvent per 1 g of thiol (1) is preferably 1 mL or more and 15 mL or less. The concentration of the trialkylaluminum-containing solution is preferably 0.1 mol/L or more and 5 mol/L or less.

As the first reaction solvent, at least one selected from the group consisting of acetonitrile, propionitrile, tetrahydrofuran (THF), 2-methyl-tetrahydrofuran, 1,4-dioxane, tert-butyl methyl ether, diisopropyl ether, dimethyloxyethane, diglyme, acetone, methyl ethyl ketone, diethyl ketone, methyl acetate, ethyl acetate, butyl acetate, methylene chloride, chloroform, carbon tetrachloride, 1,2-dichloroethane, chlorobenzene, toluene, xylene, hexane and heptane, is used. As the first reaction solvent, preferably methylene chloride, toluene, hexane or a mixed solvent of these, and more preferably methylene chloride is used.

The hydroxyl group-containing compound (II-i) obtained by the reaction between the lactone derivative (V) and the thiol (1) is preferably isolated in accordance with the following method.

First of all, a quench solution such as ice-cold water is added to the reaction solution to terminate the reaction. Then, to the reaction solution containing the quench solution, Bronsted acid is preferably added. By the addition, cyclization of the hydroxyl group-containing compound (II-i) into the structure of the lactone derivative (V) can be suppressed. The present inventors have found that particularly a hydroxyl group-containing compound (II-i) obtained by using as a substrate, a lactone derivative (V) wherein the number of atoms constituting the lactone derivative (V) is equivalent to or smaller than the number of atoms constituting a 5-membered ring, is likely to change to the structure of the substrate, compared to a hydroxyl group-containing compound obtained by using as a substrate, a lactone derivative (V) wherein the number of atoms constituting the lactone derivative (V) is equivalent to or larger than the number of atoms constituting a 6-membered ring. To overcome such a problem, the present inventors have found that the cyclization of the hydroxyl group-containing compound (II-i) is suppressed by controlling the pH of the reaction solution to be acidic, with the result that a yield thereof can be enhanced.

The amount of the Bronsted acid is, for example, 1 mol or more, preferably 3 mol or more, and more preferably 5 mol or more, relative to 1 mol of the lactone derivative (V). The upper limit of the amount of the Bronsted acid is not particularly limited but, for example, 30 mol or less.

As the Bronsted acid, at least one selected from the group consisting of hydrogen halide, sulfuric acid (H₂SO₄), carbonic acid, acetic acid, oxalic acid, citric acid, trifluoro acetic acid, methanesulfonic acid, trifluoromethanesulfonic acid, p-toluene sulfonic acid and phosphoric acid, is used. As the hydrogen halide, for example, hydrogen fluoride (HF), hydrogen chloride (HCl), hydrogen bromide (HBr) or hydrogen iodide (HI) is used. As the Bronsted acid, preferably at least one selected from the group consisting of hydrogen chloride, hydrogen bromide and sulfuric acid, is used. An acidic solution prepared by dissolving the Bronsted acid in water may be used.

In cases where a 1 N hydrochloric acid is used as the Bronsted acid, the amount thereof is preferably 10 mL or more and 30 mL or less relative to 1 g of the lactone derivative (V).

Next, the reaction solution containing the Bronsted acid is stirred to allow the solution to separate into an aqueous layer and an organic layer. After the organic layer is extracted, the same type of solvent as the solvent mentioned as the first reaction solvent is added to the aqueous layer to separate the aqueous layer again into an organic layer and an aqueous layer. The organic layer is extracted and combined with the organic layer previously extracted to obtain a total organic layer. The total organic layer is washed with, e.g., water and brine, and thereafter, dried over, e.g., sodium sulfate, to obtain a residue containing as a product the hydroxyl group-containing compound (II-i).

The structure of the hydroxyl group-containing compound (II-i) thus obtained can be confirmed, for example, by nuclear magnetic resonance (NMR) spectroscopy.

Then, the hydroxyl group of the hydroxyl group-containing compound (II-i) thus obtained is protected to obtain a thioester derivative (II-ii). The method for protecting the hydroxy group is not particularly limited and a known method can be used, which is, for example, a method for introducing a hydroxyl protecting group R⁵ by reacting the hydroxyl group-containing compound (II-i) and a protecting group introducing reagent in the presence of an acid or basic reagent in an inert solvent. The reaction is preferably carried out in an atmosphere of an inert gas such as argon.

The protecting group introducing reagent can be appropriately determined depending on the type of R⁵. Examples of the protecting group introducing reagent include: agents for introducing an ester-type protecting group, such as acetic anhydride, pivalic anhydride, acetyl chloride and pivaloyl chloride; agents for introducing an aryl alkyl ether-type protecting group, such as benzyl bromide; agents for introducing an alkyl ether-type protecting group, such as iodomethane; agents for introducing a silyl-type protecting group, such as trimethylsilyl chloride, triisopropylsilyl chloride, tert-butyldimethylsilyl chloride and tert-butyldiphenylsilyl chloride; and agents for introducing an oxycarbonyl-type protecting group, such as bis(tert-butyloxycarbonyloxy)oxide. The agents for introducing an ester-type protecting group, such as acetic anhydride, pivalic anhydride, acetyl chloride and pivaloyl chloride, are preferable and acetic anhydride is more preferable.

Examples of the acidic reagent include inorganic acids such as acetic acid and hydrogen bromide and organic acids such as p-toluenesulfonic acid and phthalic acid. Examples of the basic reagent include organic amines such as triethylamine, 4-dimethylaminopyridine (DMAP), diazabicycloundecene (DBU) and diethylaniline. Triethylamine, 4-dimethylaminopyridine (DMAP) or a mixture of these is preferable.

Although the amount of the acidic reagent used is not particularly limited, the amount thereof is, for example, 0.1 mol or more and 1000 mol or less, and preferably 1 mol or more and 5 mol or less, relative to 1 mol of the lactone derivative (V). Although the amount of the basic reagent used is not particularly limited, the amount thereof is, for example, 0.001 mol or more and 10 mol or less, and preferably 0.01 mol or more and 2 mol or less, relative to 1 mol of the lactone derivative (V).

The solvent used is preferably an organic solvent. Examples of the solvent include: polar aprotic solvents such as acetonitrile, propionitrile, THF, 2-methyl-tetrahydrofuran, 1,4-dioxane, tert-butyl methyl ether, diisopropyl ether, dimethyloxyethane, diglyme, acetone, methyl ethyl ketone, diethyl ketone, methyl acetate, ethyl acetate and butyl acetate; non-polar solvents such as methylene chloride, chloroform, carbon tetrachloride, 1,2-dichloroethane, chlorobenzene, toluene, xylene, hexane and heptane; and a combination of these. Methylene chloride, toluene or a mixed solvent of these is preferable.

Although the amount of the solvent used is not particularly limited, the amount thereof is, for example, 1 to 1000 mL, and preferably 1 to 100 mL, relative to 1 g of the lactone derivative (V).

Although the reaction temperature is not particularly limited, the reaction temperature is usually -30 to 100°C, preferably -30 to 40°C, more preferably -10 to 40°C, and still more preferably 0 to 30°C.

The thioester derivative (II-ii) obtained by introducing a hydroxyl protecting group R⁵ into the hydroxyl group-containing compound (II-i) is preferably isolated in accordance with the following method.

First of all, a quench solution such as water is added to a reaction solution to terminate the reaction. The reaction solution containing the quench solution is stirred to allow the solution to separate into an aqueous layer and an organic layer. After the organic layer is extracted, the same type of solvent as the solvent mentioned as the first reaction solvent is added to the aqueous layer to separate the aqueous layer again into an organic layer and an aqueous layer. The organic layer is extracted and combined with the organic layer previously extracted to obtain a total organic layer. The total organic layer is washed with, e.g., water and brine, and thereafter, dried over, e.g., sodium sulfate, to obtain a residue containing as a product the thioester derivative (II-ii).

The structure of the thioester derivative (II-ii) thus obtained can be confirmed, for example, by nuclear magnetic resonance (NMR) spectroscopy.

### <Grignard reagent (III)>

The Grignard reagent (III) is selected from a Grignard reagent (IIIa) represented by the following formula (IIIa):

W²MgX (IIIa)

and a Grignard reagent (IIIb) represented by the following formula (IIIb):

W²MgX·LiCl (IIIb)

In formulas (IIIa) and (IIIb), W² is the same as defined above, and X represents a halogen atom. The halogen atom is preferably selected from a chlorine atom, a bromine atom and an iodine atom.

As the Grignard reagent (III), either one or both of the Grignard reagent (IIIa) and the Grignard reagent (IIIb) may be selected. In cases where both Grignard reagents are selected, a mixture of both of the Grignard reagents may be added to a reaction system or the Grignard reagents may be separately added to the reaction system.

The Grignard reagent (IIIa) may be a commercially available compound or produced by a conventional method.

In view of improvement of a reaction rate, the Grignard reagent (IIIb) is preferably included as the Grignard reagent (III). Note that, the Grignard reagent (IIIb) is called a turbo Grignard reagent.

The Grignard reagent (IIIb) may be a commercially available compound or produced by a conventional method. The Grignard reagent (IIIb) can be produced, by reacting magnesium and a halogen organic compound represented by formula: W²X [wherein W² and X are the same as defined above] in an organic solvent in the presence of a lithium salt in a reaction vessel purged with, for example, an inert gas (for example, nitrogen, argon).

The Grignard reagent (IIIb) may be produced by reacting a Knochel-Hauser base represented by formula: TMPMgX·LiY [wherein TMP represents 2,2,6,6-tetramethylpiperidine] and a compound represented by formula: W²-H, in accordance with a known method and described in, e.g., Angew Chem. Int. Ed2006, 45, 2958.

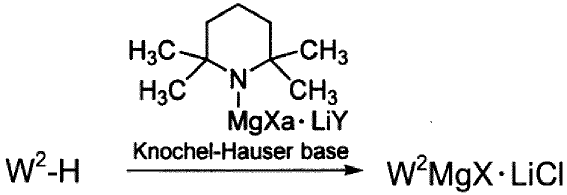

### <Copper Salt>

Examples of the copper salt include copper (I) chloride (CuCl), copper (II) chloride (CuCl₂), copper (I) bromide (CuBr), copper (II) bromide (CuBr₂), copper (I) cyanide (CuCN), 3-copper (I) methylsalicylate, mesitylene copper (I) (MesCu), isopropoxy copper (I) (ⁱPrOCu), copper iodide (I) (CuI), copper (II) iodide (CuI₂), copper (I) acetate (CuOAc), copper (II) acetate (Cu(OAc)₂), copper (II) sulfate (CuSO₄), copper (I) oxide (Cu₂O), copper (II) oxide (CuO), copper (I) pivalate (CuOPiv), copper (II) pivalate (Cu(OPiv)₂) and a sulfur (S)-containing copper salt. The valence number of a copper atom contained in a copper salt is usually one or two, preferably, one. A copper salt wherein the valence number of a copper atom is one has an excellent catalytic action. Of the copper salts wherein the valence number of a copper atom is one, CuCl, CuI and CuBr are particularly preferable. CuCl, CuI and CuBr have particularly excellent catalytic action. Examples of the sulfur (S)-containing copper salt include copper(I) thiophene-2-carboxylate. Since S has a high affinity for Cu, S is easily coordinated with Cu in a copper salt. Cu is activated by the coordination to increase a yield.

### <Method for producing a ketone derivative (I)>

A method for producing a ketone derivative (I) includes a step of mixing a thioester derivative (II), a Grignard reagent (III) and a copper salt to form a ketone derivative (I).

The ketone derivative (I) can be obtained with a high yield by mixing the thioester derivative (II), the Grignard reagent (III) and the copper salt. The present inventors presume that this is because an anionic complex represented by the following formula (10) is formed, more specifically, because the oxidative addition of the carbon-sulfur bond of the thioester derivative (II) is accelerated not to a neutral complex but to an anionic complex (10).

[W²-Cu-W²]⁻ (10)

When the thioester derivative (II), the Grignard reagent (III) and the copper salt are mixed, it is preferable that the Grignard reagent (III) and the copper salt are mixed to form an organic copper reagent (organocuprate reagent), and then, the thioester derivative (II) is mixed to allow the organic copper reagent and the thioester derivative (II) to be in contact. In this manner, the ketone derivative (I) can be obtained with a high yield.

The amount of the copper salt used is preferably 0.1 mol or more and 1 mol or less relative to 1 mol of the Grignard reagent (III). In cases where the amount of the copper salt used relative to the Grignard reagent (III) to fall within this range, formation of the above anionic complex (10) tends to smoothly proceed. The amount of the copper salt used is more preferably 0.3 mol or more and 0.9 mol or less, and still more preferably 0.4 mol or more and 0.8 mol or less, relative to 1 mol of the Grignard reagent (III). The amount of the copper salt used is 0.5 mol or more and 0.9 mol or less relative to 1 mol of the Grignard reagent (III) in an embodiment, and 0.6 mol or more and 0.8 mol or less relative to 1 mol of the Grignard reagent (III) in another embodiment.

In cases where the Grignard reagent (IIIb) is included as the Grignard reagent (III), the amount of the copper salt used is usually 0.1 mol or more and 1 mol or less, preferably 0.3 mol or more and 0.9 mol or less, and more preferably 0.4 mol or more and 0.8 mol or less, relative to 1 mol of the Grignard reagent (IIIb). Alternatively, in cases where the Grignard reagent (IIIb) is included as the Grignard reagent (III), the amount of the copper salt used is 0.4 mol or more and 0.92 mol or less relative to 1 mol of the Grignard reagent (IIIb) in an embodiment, 0.5 mol or more and 0.82 mol or less relative to 1 mol of the Grignard reagent (IIIb) in another embodiment, and 0.6 mol or more and 0.72 mol or less relative to 1 mol of the Grignard reagent (IIIb) in yet another embodiment.

The amount of the copper salt used is usually 0.1 mol or more and 10 mol or less, preferably 0.5 mol or more and 5 mol or less, more preferably 0.6 mol or more and 3 mol or less, and still more preferably 1 mol or more and 3 mol or less, relative to 1 mol of the thioester derivative (II).

The amount of the Grignard reagent (III) used is usually 1 mol or more and 10 mol or less, preferably 1 mol or more and 5 mol or less, more preferably 1.05 mol or more and 4 mol or less, and still more preferably 1.5 mol or more and 4 mol or less, relative to 1 mol of the thioester derivative (II). The amount of the Grignard reagent (III) used is not necessary to exceed the amount of the thioester derivative (II) used.

In cases where the Grignard reagent (IIIa) and the Grignard reagent (IIIb) are both selected as the Grignard reagent (III), the amount of the Grignard reagent (IIIb), for example, is 10 to 90 mass% based on the total mass of the Grignard reagent (IIIa) and the Grignard reagent (IIIb).

Examples of the solvent used in mixing the thioester derivative (II), the Grignard reagent (III) and the copper salt include tetrahydrofuran (THF), 2-methyl-tetrahydrofuran, 1,4-dioxane, tert-butyl methyl ether, cyclopentyl methyl ether, dimethoxyethane, diglyme, methylene chloride, toluene, xylene, hexane and heptane. A single solvent may be used alone or two or more solvents may be used in combination as a mixed solvent. The solvent is preferably THF, toluene or a mixed solvent of these.

The amount of the solvent used is usually 1 to 100 mL, and preferably 2 to 50 mL, relative to 1 g of the thioester derivative (II).

The temperature at which the thioester derivative (II), the Grignard reagent (III) and the copper salt are mixed is usually within the range of - 10°C or more and 100°C or less. In the method according to an embodiment, the Grignard reagent (III) is used, and thus the ketone derivative (I) can be produced even under relatively high temperature conditions. Accordingly, compared to a method requiring an ultralow temperature condition lower than -10°C for synthesizing the ketone derivative (I), the method of the invention can suppress the cost of equipment for temperature control, and thus realize more inexpensive industrial production of the ketone derivative (I). The temperature for mixing is preferably within the range of 10°C or more and 80°C or less, and more preferably 20°C or more and 60°C or less. In cases where the temperature falls within the range, the yield of the ketone derivative (I) tends to increase.

The time for mixing the thioester derivative (II), the Grignard reagent (III) and the copper salt is usually 0.5 to 72 hours, and preferably 1 to 48 hours.

When the thioester derivative (II), the Grignard reagent (III) and the copper salt are mixed, either one or both of
an organic zinc compound (III-I) represented by the following formula (III-I):

   W²ZnX (III-I)
wherein W² and X are the same as defined above, and
an organic zinc compound (III-II) represented by the following formula (III-II):

   (W²)₂Zn (III-II)
wherein W² is the same as defined above,
   may be used in combination with the Grignard reagent (III) and the copper salt. The organic zinc compound (III-I) and the organic zinc compound (III-II) can be used as reagents for introducing a group W² into the thioester derivative (II).

However, when the thioester derivative (II), the Grignard reagent (III) and the copper salt are mixed, it is preferable that neither the organic zinc compound (III-I) nor the organic zinc compound (III-II) is used as much as possible. In cases where the organic zinc compound (III-I) and the organic zinc compound (III-II) are contained, the yield of the ketone derivative (I) tends to decrease. The total amount of the organic zinc compound (III-I) and the organic zinc compound (III-II) used is preferably 10 mass% or less, more preferably 5 mass% or less, and still more preferably 1 mass% or less, based on the mass of the thioester derivative (II). The lower limit thereof is zero.

Examples of the organic zinc compound (III-I) include an aryl zinc halide (a compound represented by formula (III-I) wherein W² is an aryl group, X is a halogen atom, preferably a chlorine atom, a bromine atom or an iodine atom) and an alkyl zinc halide (a compound represented by formula (III-I) wherein W² is an alkyl group, X is a halogen atom, preferably a chlorine atom, a bromine atom or an iodine atom).

Examples of the organic zinc compound (III-II) include a diaryl zinc (a compound represented by formula (III-II) wherein W² is an aryl group), a dialkyl zinc (a compound represented by formula (III-II) wherein W² is an alkyl group).

The organic zinc compound (III-I) and the organic zinc compound (III-II) may be commercially available compounds or produced by a conventional method.

The organic zinc compound (III-I) and/or the organic zinc compound (III-II) may be used together with a lithium salt such as lithium chloride. The organic zinc compound (III-I) may form a complex with a lithium salt. The complex of the organic zinc compound (III-I) and the lithium salt can be represented, for example, by the following formula (III-Ia).

W²ZnX·LiCl (III-Ia)

In formula (III-Ia), W² and X are the same as defined above.

### <Grignard reagent (IV)>

In cases where W² is an aryl group in which a carbon atom adjacent to each of two sides of a carbon atom having a bond of the aryl group (a carbon atom binding to W¹-CO- or W²-CO- in formula (I); a carbon atom binding to Mg in formula (IIIa) or (IIIb)) has no substituent, and the remaining carbon atoms may have a substituent; or is a heteroaryl group in which a carbon atom or heteroatom adjacent to each of two sides of a carbon atom having a bond of the heteroaryl group (a carbon atom binding to W¹-CO- or W²-CO- in formula (I); a carbon atom binding to Mg in formula (IIIa) or (IIIb)) has no substituent and the remaining carbon atoms or heteroatom(s) may have a substituent, a Grignard reagent (IV) represented by the following formula (IV):

W⁴MgX¹ (IV)

may be used in the method for producing the ketone derivative (I).

In formula (IV), W⁴ represents a phenyl group that has a substituent(s) at at least one of the ortho-positions and that may have a substituent(s) at the meta-position(s) and/or para-position, and X¹ represents a halogen atom.

The Grignard reagent (IV) can be used together with either one or both of the Grignard reagents (IIIa) and (IIIb). In this case, a mixture of either one or both of the Grignard reagents (IIIa) and (IIIb) and the Grignard reagent (IV) may be added to a reaction system or either one or both of the Grignard reagents (IIIa) and (IIIb) and the Grignard reagent (IV) are separately added to a reaction system.

In addition to the Grignard reagent (III) and the Grignard reagent (IV), another Grignard reagent may be used. In this case, the total amount of the Grignard reagent (III) and the Grignard reagent (IV) is preferably 80 mass% or more or may be 100 mass%, based on the total amount of the Grignard reagent (III), the Grignard reagent (IV) and the other Grignard reagent.

In cases where the Grignard reagent (IV) is used, the thioester derivative (II), the Grignard reagent (III), the copper salt and the Grignard reagent (IV) are mixed to form the ketone derivative (I).

When the thioester derivative (II), the Grignard reagent (III), the copper salt and the Grignard reagent (IV) are mixed, it is preferable that the Grignard reagent (III) and the copper salt are mixed, and thereafter the Grignard reagent (IV) is mixed to form an organic copper reagent (organocuprate reagent), and thereafter the thioester derivative (II) is mixed to allow the organic copper reagent and the thioester derivative (II) to be in contact with each other. In this manner, the ketone derivative (I) can be obtained with a high yield.

A compound represented by the following formula (III') is an example of the Grignard reagent (IIIa) or (IIIb) wherein W² is an aryl group in which a carbon atom adjacent to each of two sides of a carbon atom having a bond of the aryl group (a carbon atom binding to Mg) has no substituent and the remaining carbon atoms may have a substituent; or is a heteroaryl group in which a carbon atom or heteroatom adjacent to each of two sides of a carbon atom having a bond of the heteroaryl group (a carbon atom binding to Mg) have no substituent and the remaining carbon atoms or heteroatom(s) may have a substituent.

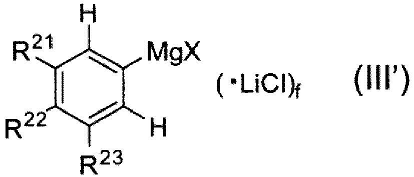

In formula (III'), the carbon atom adjacent to each of two sides of the carbon atom binding to MgX, in other words, the carbon atom at each of the ortho-positions has no substituent. R²¹ and R²³ are present the meta-positions relative to the carbon atom binding to MgX. R²² is present at the para-position of the carbon atom binding to MgX. R²¹, R²² and R²³ are each independently a hydrogen atom or a substituent selected from substituent groups α and β. f is 0 or 1.

A compound represented by the following formula (IV') is an example of the Grignard reagent (IV). Now, W⁴ will be described more specifically, below.

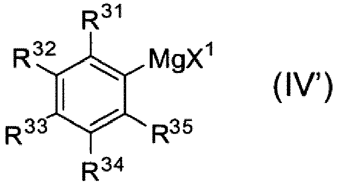

In formula (IV'), R³¹, R³², R³³, R³⁴ and R³⁵ each independently represent a hydrogen atom or a substituent. At least one of R³¹ and R³⁵ binding to the meta-positions relative to the carbon atom binding to MgX¹ is a substituent. Both of R³¹ and R³⁵ are preferably substituents.

Examples of the substituent include an alkyl group, an arylalkyl group, a halogen group, a nitrile group, a dialkylamino group, an alkyloxy group, an arylalkyloxy group, an alkylthio group and an arylalkylthio group. The number of carbon atoms of each of the alkyl group, alkyloxy group and alkylthio group is preferably 1 to 10. The number of carbon atoms of the dialkylamino group is preferably 2 to 10. The number of carbon atoms of each of the arylalkyl group, arylalkyloxy group and arylalkylthio group is preferably 5 to 14, and more preferably 7 to 14.

The substituent is preferably an alkyl group, and more preferably a methyl group. Preferable examples of W⁴ include a 2,4,6-trimethylphenyl group and a 2,6-dimethylphenyl group.

According to the method using the Grignard reagent (IV), the yield of the ketone derivative (I) can be increased. The present inventors presume the reason for this as follows: The Grignard reagents (IIIa) and (IIIb) each are a compound wherein W² is an aryl group in which a carbon atom adjacent to each of two sides of a carbon atom binding to Mg has no substituent or is a heteroaryl group in which a carbon atom or heteroatom adjacent to each of two sides of a carbon atom binding to Mg has no substituent. The Grignard reagent (IV) is a compound wherein W⁴ is a phenyl group in which at least one of two carbon atoms adjacent to two sides of a carbon atom binding to Mg has a substituent. In cases where the Grignard reagent (IIIa) and/or (IIIb) and the Grignard reagent (IV) are used, an anionic complex represented by the following formula (11) is formed. In the anionic complex (11), W⁴ has a substituent(s) at at least one of the ortho-positions, and thus steric hindrance occurs, thereby inhibiting the substitution reaction from -SW³ to -W⁴. As a result, the substitution reaction from -SW³ to -W² is accelerated. Thus, compared to the case where the above complex (10) is used, the yield of the ketone derivative (I) per amount of the Grignard reagents (IIIa) and (IIIb) used is improved. In addition, the reactivity of W² can be improved by the substituent(s) at the ortho-position(s) due to electron donating effect. Furthermore, by introducing W⁴ that is not subjected to a reaction, in a reactant (organic copper reagent), it is possible to reduce the initial amount of a Grignard reagent generating W² actually involved in a reaction.

[W²-Cu-W⁴]⁻ (11)

In the method using the Grignard reagent (IV), the substitution reaction from -SW³ to -W² in the thioester derivative (II) can be accelerated, and thus the amounts of the copper salt and the Grignard reagent (III) can be comparatively reduced.

The amount of the copper salt used is, for example, 0.1 mol or more and 10 mol or less, preferably 0.5 mol or more and 5 mol or less, and more preferably 0.5 mol or more and 2 mol or less, relative to 1 mol of the Grignard reagent (III).

The amount of the copper salt used is, for example, 0.1 mol or more and 10 mol or less, preferably 0.5 mol or more and 5 mol or less, and more preferably 0.5 mol or more and 2 mol or less, relative to 1 mol of the Grignard reagent (IV). In another embodiment, the amount of the copper salt used is 1 mol or more and 3 mol or less relative to 1 mol of the Grignard reagent (IV).

The amount of the copper salt used is, for example, 0.1 mol or more and 10 mol or less, preferably 0.5 mol or more and 5 mol or less, and more preferably 0.5 mol or more and 1.4 mol or less, relative to 1 mol of the thioester derivative (II).

The amount of the Grignard reagent (III) used is, for example, 0.1 mol or more and 10 mol or less, preferably 0.5 mol or more and 5 mol or less, and more preferably 0.5 mol or more and 1.4 mol or less, relative to 1 mol of the thioester derivative (II).

The amount of the Grignard reagent (IV) used is, for example, 0.01 mol or more and 1 mol or less, preferably 0.01 mol or more and 0.8 mol or less, and more preferably 0.1 mol or more and 0.8 mol or less, relative to 1 mol of the Grignard reagent (III).

The amount of the Grignard reagent (IV) used is, for example, 0.1 mol or more and 10 mol or less, preferably 0.1 mol or more and 5 mol or less, and more preferably 0.1 mol or more and 1.0 mol or less, relative to 1 mol of the thioester derivative (II).

The reaction solvent and reaction conditions are the same as defined in the production method for the ketone derivative (I).

Now, the cases where the thioester derivative (IIa) is used as the thioester derivative (II) will be described.

In the cases where the thioester derivative (IIa) is used as the thioester derivative (II), the thioester derivative (IIa), the Grignard reagent (III) and the copper salt are mixed to obtain the ketone derivative (Ia). The reaction scheme is as follows.

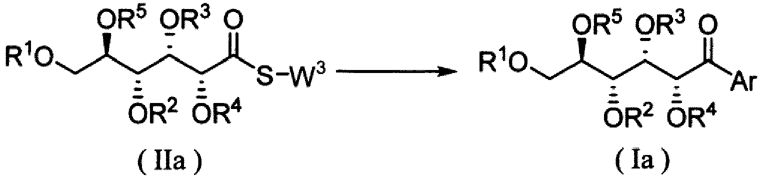

From the ketone derivative (Ia) obtained, the hydroxy group protecting group represented by R⁵ is removed to obtain a compound (IVa) represented by the following formula (IVa).

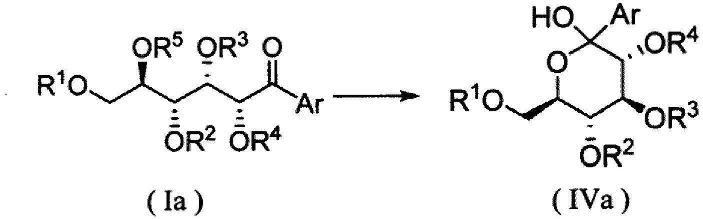

The hydroxy group protecting group represented by R⁵ can be removed in accordance with a conventional method, which varies depending on the type of hydroxy group protecting group. For example, the hydroxy group protecting group represented by R⁵ can be removed by reacting the ketone derivative (Ia), an acidic reagent or a basic reagent in an inert solvent. Examples of the acidic reagent include inorganic acids such as hydrochloric acid, sulfuric acid, nitric acid, acetic acid and hydrogen bromide; and organic acids such as trifluoroacetic acid, trichloroacetic acid, p-toluenesulfonic acid, formic acid and phthalic acid. Examples of the basic reagent include fluorides such as tetra-n-butylammonium fluoride, ammonium fluoride, ammonium bifluoride and hydrofluoric acid; potassium carbonate, lithium hydroxide, sodium hydroxide, potassium hydroxide, sodium methoxide, sodium ethoxide and ammonia water.

After the thioester derivative (IIa), the Grignard reagent (III) and the copper salt are mixed to form the ketone derivative (Ia), the ketone derivative (Ia) may be separated from the reaction system and reacted with the acidic reagent or the basic reagent to obtain the compound (IVa). Alternatively, after the thioester derivative (IIa), the Grignard reagent (III) and the copper salt are mixed to form the ketone derivative (Ia), the acidic reagent or the basic reagent may be added to the reaction system without separating the ketone derivative (Ia) from the reaction system, to obtain the compound (IVa). In the latter case, since it is not necessary to separate the ketone derivative (Ia) from the reaction system, the compound (IVa) can be efficiently obtained. Now, reaction conditions of the latter case will be described. The solvent used in mixing the thioester derivative (IIa), the Grignard reagent (III) and the copper salt is preferably, e.g., tetrahydrofuran (THF). The basic reagent is preferably used. The basic reagent is preferably, e.g., sodium methoxide, sodium ethoxide, potassium carbonate, sodium carbonate, sodium hydroxide or ammonia. The amount of the basic reagent used is usually 0.001 mol or more and 10 mol or less, and preferably 0.01 mol or more and 8 mol or less, relative to 1 mol of the ketone derivative (Ia). The amount of the solvent used is usually 1 to 500 mL or less, and preferably 3 to 200 mL, relative to 1 g of the ketone derivative (Ia). The temperature at which the ketone derivative (Ia) and the basic reagent are reacted is usually -20 to 120°C, and preferably -10 to 100°C. The time for reacting the ketone derivative (Ia) and the basic reagent is usually 0.1 to 48 hours and preferably 0.5 to 24 hours.

The thioester derivative (IIa) may be a commercially available product or produced in accordance with the following reaction scheme.

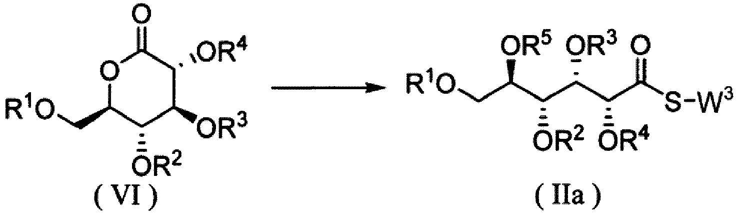

The thioester derivative (IIa) can be obtained by
reacting a compound (VI) represented by the following formula (VI): 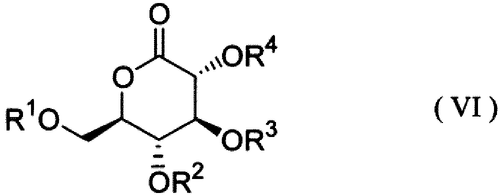 and a compound (VII) represented by the following formula (VII):

   aSH (VII)

   to obtain a compound (VIII) represented by the following formula (VIII): 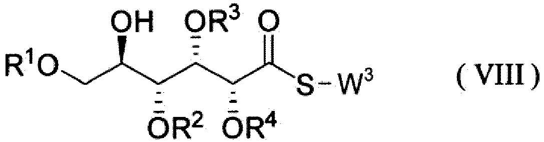 and
protecting the hydroxy group of the compound (VIII) with a hydroxy group protecting group represented by R⁵.

The compound (VI) and the compound (VII) may be commercially available products or produced in accordance with conventional methods.

A hydroxy group protecting group can be introduced to the hydroxy group of the compound (VIII) in accordance with a conventional method, which varies depending on the type of hydroxy group protecting group. For example, the compound (VIII) is reacted with a protecting group introducing reagent in an inert solvent, in the presence of an acid or basic reagent to introduce a hydroxy group protecting group. Examples of the protecting group introducing reagent include: agents for introducing an ester-type protecting group, such as acetic anhydride, pivalic anhydride, acetyl chloride and pivaloyl chloride; agents for introducing an aryl alkyl ether type protecting group, such as benzyl bromide; agents for introducing an alkyl ether type protecting group, such as iodomethane; agents for introducing a silyl-type protecting group, such as trimethylsilyl chloride, N-trimethylsilylimidazole, triisopropylsilyl chloride, tert-butyldimethylsilyl chloride and tert-butyldiphenylsilyl chloride; and agents for introducing an oxycarbonyl-type protecting group, such as bis(tert-butyloxycarbonyloxy)oxide. The agents for introducing an ester-type protecting group, such as acetic anhydride, pivalic anhydride, acetyl chloride or pivaloyl chloride, are preferable, and acetic anhydride is more preferable. Examples of the acidic reagent include inorganic acids such as acetic acid and hydrogen bromide and organic acids such as p-toluenesulfonic acid and phthalic acid. Examples of the basic reagent include organic amines such as triethylamine, 4-dimethylaminopyridine (DMAP), diazabicycloundecene (DBU) and diethylaniline.

The reaction between the compound (VI) and the compound (VII) is preferably carried out in the presence of a compound (IX) represented by the following formula (IX). The compound (IX) may be a commercially available aluminum catalyst or produced in accordance with a conventional method.

Al(R^{c})_{q}(R^{d})ᵣ (IX)

In formula (IX), R^{c} and R^{d} each independently represent a halogen atom, an alkyl group that may have a substituent, a heterocycloalkyl group that may have a substituent, an aryl group that may have a substituent, a heteroaryl group that may have a substituent, an arylalkyl group that may have a substituent or a heteroarylalkyl group that may have a substituent. Preferably, R^{c} and R^{d} each independently are an alkyl group, an aryl group or an arylalkyl group.

In formula (IX), "q" represents an integer of 0 to 3; and "r" represents an integer of 0 to 3, with the proviso that "q" + "r" = 3. In a preferable embodiment, either one of "q" and "r" represents 0 and the other represents 3.

Using the compound (IVa) as a starting material, a compound (XI) represented by the following formula (XI), i.e., a β-C-aryl glycoside derivative, can be produced.

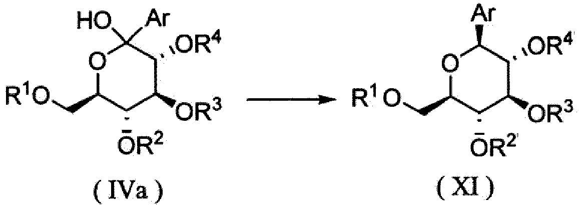

As the reaction for producing the compound (XI) from the compound (IVa), a known reduction reaction can be used. Examples of the reduction method include a reducing method using triethylsilane in the presence of a boron trifluoride diethyl ether complex (BF₃·OEt₂); and a method by reacting with a Lewis acid such as BF₃·OEt₂, boron trifluoride tetrahydrofuran (BF₃·THF) or aluminum chloride in the presence of a silane compound such as triethylsilane, triisopropylsilane or tetramethyldisiloxane.

The compound (XI) thus obtained can be directly used as a β-C-aryl glycoside derivative or after deprotection, if desired, when R¹, R², R³ or R⁴ is a hydroxy group protecting group.

### EXAMPLES

### <Example 1>

The reaction shown in the following scheme was carried out to produce compound 2 from compound 1. Note that, "Ph" stands for a phenyl group. The same applies hereinafter.

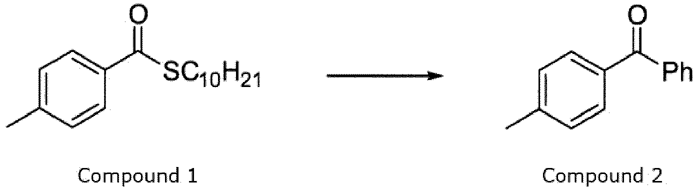

To a suspension of CuI (35.8 mg, 0.188 mmol, 0.75 equivalents) in dry THF (2 mL), a solution of 0.85 M PhMgBr in THF (0.330 mL, 0.281 mmol, 1.1 equivalents) was added dropwise over 5 minutes and the mixture was stirred for 10 minutes. In this stage, an organic copper reagent was produced. To the organic copper reagent produced, a solution of S-decyl 4-methylbenzothioate (73.1 mg, 0.250 mmol, 1.0 equivalent) in THF (2 mL) was added dropwise over 5 minutes, and thereafter, the reaction mixture was stirred at 25°C for one hour. After completion of the reaction, the reaction mixture was quenched with a 1 N aqueous HCl solution (1 mL). To the reaction mixture, a predetermined amount of a solution of triphenylmethane (internal standard substance) in ethyl acetate (3 mL) was added. The reaction mixture was stirred and then subjected to short pad silica gel filtration (ethyl acetate) to prepare a sample for gas chromatography. The yield was obtained by gas chromatographic analysis.

Gas chromatographic analysis conditions are as follows. - Apparatus name: GC-2014 (SHIMADSU GAS CHROMATOGRAPH), - Column: SH-Rtx-50 (length: 30.0 mm, inner diameter: 0.25 mm, film thickness: 0.25 µm, medium polar column)

### - Analysis conditions

Injection volume: 1.0 µL
Column-oven temperature program (total time of program: 15 minutes)

**[Table 2]**

| | Rate (°C/min) | Temperature (°C) | Hold Time (min) |
|---|---|---|---|
| 0 | - | 100.0 | 1.00 |
| 1 | 40.00 | 300.0 | 9.00 |

**[Table 3]**

| Hold Time (min) | |
|---|---|
| Substance | Hold Time (min) |
| Biphenyl | 6.4-6.5 |
| 4-Methylbenzophenone | 8.2-8.3 |
| Triphenylmethane (internal standard substance) | 9.6-9.7 |
| S-Decyl 4-Methylbenzothioate | 10.7-10.8 |
| 4-Methyltriphenylcarbinol | 12.2-12.3 |

Yields were obtained by using triphenylmethane (manufactured by Aldrich, 25 g) as an internal standard substance and based on integral ratios. The results are shown in Table 4. The yield of compound 2 in Example 1 was 89%.

Physical properties of compound 2 were as follows.
¹H NMR (400 MHz, CDCl₃, 30°C) δ 7.80-7.77 (m, 2H), 7.73-7.71 (m, 2H), 7.59-7.55 (m, 1H), 7.49-7.45 (m, 2H), 7.29-7.27 (m, 2H), 2.44 (s, 3H). ¹³C NMR (100 MHz, CDCl₃, 30°C) δ 196.6, 143.4, 138.2, 135.1, 132.3, 130.5, 130.1, 129.1, 128.4, 21.8.

### <Example 2>

The same operation as in Example 1 was repeated except that the amount of CuI was changed to 0.25 equivalents. The results are shown in Table 4. The yield of compound 2 in Example 2 was 48%.

### <Example 3>

The same operation as in Example 1 was repeated except that the amount of CuI was changed to 0.50 equivalents. The results are shown in Table 4. The yield of compound 2 in Example 3 was 63%.

### <Example 4>

The same operation as in Example 1 was repeated except that the amount of CuI was changed to 1.0 equivalent. The results are shown in Table 4. The yield of compound 2 in Example 4 was 33%.

### <Comparative Example 1>

The same operation as in Example 1 was repeated except that CuI was not used. The results are shown in Table 4. The yield of a ketone derivative (1) in Comparative Example 1 was 0%.

**[Table 4]**

| 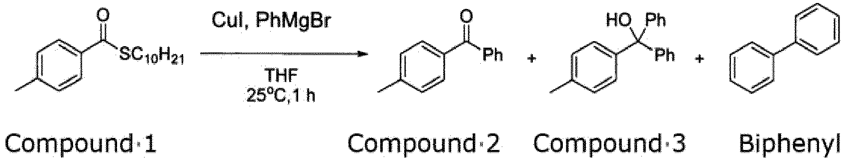 | | | | |
|---|---|---|---|---|
| | Amount of CuI relative to 1 mol of PhMgBr | | | |
| Example 1 | 0.67 mol | | | |
| Example 2 | 0.22 mol | | | |
| Example 3 | 0.45 mol | | | |
| Example 4 | 0.89 mol | | | |
| Comparative Example 1 | 0 | | | |

| | Yield (%) | | | Recovery Rate of Compound 1 (%) |
|---|---|---|---|---|
| | Compound 2 | Compound 3 | Biphenyl | |
| Example 1 | 89 | < 5 | approx. 8 | 8 |
| Example 2 | 48 | < 5 | approx. 6 | 40 |
| Example 3 | 63 | < 5 | approx. 9 | 22 |
| Example 4 | 33 | not detected | < 5 | 69 |
| Comparative Example 1 | not detected | not detected | not detected | > 95 |

### <Reference Example 1 (production of compound 1)>

The reaction shown in the following scheme was carried out to produce compound 1.

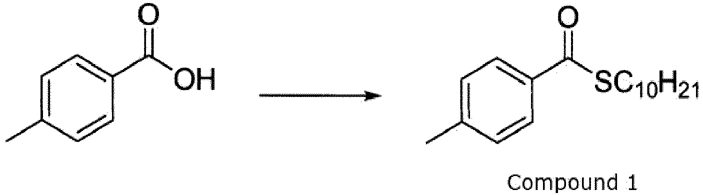

In an 80 mL Schlenk tube, a suspension of p-toluic acid (1.43 g, 10.5 mmol) in methylene chloride (20 mL) was prepared. After the suspension was cooled up to 0°C, 4-dimethylaminopyridine (122 mg, 1.00 mmol), 1-decanethiol (2.08 mL, 10.0 mmol) and N,N'-dicyclohexylcarbodiimide (2.17 g, 10.5 mmol) were added. After stirring at 0°C for 30 minutes, the temperature was raised to room temperature and the suspension was stirred overnight. After completion of the reaction, the suspension was filtered. The filtrate was washed with a 1 M aqueous HCl solution, saturated sodium bicarbonate water and saturated brine (50 mL for each × 1), dried over Na₂SO₄, and concentrated under reduced pressure. The concentrated residue was purified by silica column chromatography (n-hexane, then, ethyl acetate/n-hexane = 1:20) to obtain compound 1 (2.73 g, 93%).
¹H NMR (400 MHz, CDCl₃, 30°C) δ 7.86 (d, J = 8.1 Hz, 2H), 7.23 (d, J = 8.0 Hz, 2H), 3.05 (t, J = 7.3 Hz, 2H), 2.40 (s, 3H), 1.66 (quint, J = 7.4 Hz, 2H), 1.42 (quint, J = 7.1 Hz, 2H), 1.35-1.26 (m, 12H), 0.88 (t, J = 6.8 Hz, 3H).
¹³C NMR (100 MHz, CDCl₃, 30°C) δ 191.9, 170.1, 144.1, 135.0, 129.4, 127.4, 32.0, 29.8, 29.7, 29.66, 29.5, 29.3, 29.1, 22.8, 21.8, 14.2.
HRMS: [M+H]⁺ C₁₈H₂₉OS
Calculated: 293.1934, Found: 293.1938.

### <Reference Example 2 (synthesis of an alcohol)>

The reaction shown in the following scheme was carried out to produce an alcohol.

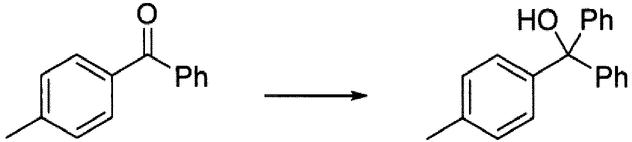

In a 20 mL Schlenk tube, a solution of 4-methylbenzophenone (196 mg, 1.00 mmol) in THF (4.0 mL) was prepared. After a solution of 0.85 M PhMgBr in THF (1.3 mL, 1.11 mmol) was added dropwise, the mixture was refluxed at 80°C for one hour. The reaction was terminated with a 1 M aqueous HCl solution (5 mL), and ethyl acetate (10 mL) was added thereto. Thereafter, the resultant organic layer was washed with a 1 M aqueous HCl solution (5 mL × 2) and saturated brine (5 mL × 1). The organic layer washed was dried over Na₂SO₄ and concentrated under reduced pressure. The concentrated residue was purified by silica column chromatography (ethyl acetate/n-hexane = 1:20 to 1:10) to obtain an alcohol (243 mg, 89%).
¹H NMR (400 MHz, CDCl₃, 30°C) δ 7.31-7.23 (m, 10H), 7.12 (q, J = 8.2 Hz, 4H), 2.76 (s, 1H), 2.33 (s, 3H).
¹³C NMR (100 MHz, CDCl₃, 30°C) δ 147.2, 144.2, 137.0, 128.7, 128.0, 127.9₉, 127.3, 82.0, 21.1.

### <Example 5>

The reaction shown in the following scheme was carried out to produce compound 5 from compound 4. Note that, "Ac" stands for an acetyl group and "Bn" stands for a benzyl group. The same applies hereinafter.

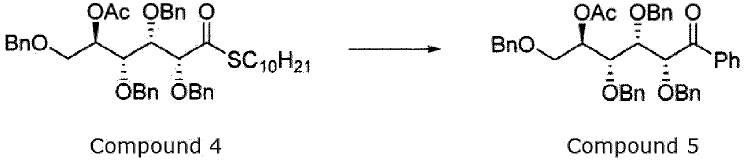

To a suspension of CuI (35.8 mg, 0.188 mmol, 0.75 equivalents) in dry THF (2 mL), a solution of 0.85 M PhMgBr in THF (0.330 mL, 0.281 mmol, 1.1 equivalents) was added dropwise over 5 minutes and the reaction mixture was stirred for 10 minutes. In this stage, an organic copper reagent was produced. To the organic copper reagent produced, a solution of a thioester derivative (189 mg, 0.250 mmol, 1.0 equivalent) in THF (2 mL) was added dropwise over 5 minutes, and thereafter, the mixture was stirred at room temperature for 20 minutes. After completion of the reaction, the reaction was quenched with a 1 N aqueous HCl solution (1 mL). To the reaction solution, an ethyl acetate (10 mL) was added and the resultant organic layer was washed with 1 N aqueous HCl solution (5 mL × 3) and saturated brine (5 mL × 1). The organic layer washed was dried over Na₂SO₄ and concentrated under reduced pressure. Thereafter the concentrated residue was purified by silica column chromatography (ethyl acetate/n-hexane = 1:20 -> 1:5) to obtain a ketone compound (compound 5)(71.8 mg, 44%); at the same time, a thioester compound (compound 4)(99.4 mg, 53%) was recovered.

Physical properties of the ketone compound (compound 5) were as follows.
¹H NMR (400 MHz, CDCl₃, 30°C) δ 7.92 (dd, J = 8.3, 1.1 Hz, 2H), 7.49-7.45 (m, 1H), 7.36-7.14 (m, 20H), 7.05-7.02 (m, 2H), 5.28 (dt, J = 5.4, 3.5 Hz, 1H), 4.89 (d, J = 4.2 Hz, 1H), 4.67-4.41 (m, 7H), 4.31 (d, J = 10.8 Hz, 1H), 4.21 (dd, J = 6.9, 4.3 Hz, 1H), 4.07 (dd, J = 6.9, 3.5 Hz, 1H), 3.87 (dd, J = 10.2, 5.4 Hz, 1H), 3.63 (dd, J = 10.2, 5.5 Hz, 1H), 1.97 (s, 3H).
¹³C NMR (100 MHz, CDCl₃, 30°C) δ 199.0, 170.1, 138.5, 138.0, 137.9, 137.2, 136.2, 133.2, 129.1, 128.9, 128.6, 128.5, 128.4, 128.36, 128.3, 128.2, 128.0, 127.9, 127.8, 127.81, 127.6, 127.56, 82.8, 80.5, 79.7, 75.4, 74.7, 73.3, 73.2, 72.7, 68.0, 21.2.

Physical properties of the thioester compound (compound 4) were as follows.
¹H NMR (400 MHz, CDCl₃, 30°C) δ 7.42-7.21 (m, 20H), 5.17-5.14 (m, 1H), 4.81-4.42 (m, 8H), 4.25 (d, J = 4.4 Hz, 1H), 4.01-3.95 (m, 2H), 3.82 (dd, J = 10.7, 4.2 Hz, 1H), 3.65 (dd, J = 10.6, 5.7 Hz, 1H), 2.84 (t, J = 7.4 Hz, 2H), 1.96 (s, 3H), 1.56 (quint, J = 7.4 Hz, 2H), 1.37-1.26 (m, 14H), 0.88 (t, J = 6.9 Hz, 3H).
¹³C NMR (100 MHz, CDCl₃, 30°C) δ 202.0, 170.1, 138.6, 138.2, 138.1, 137.2, 128.7, 128.6, 128.5, 128.46, 128.43, 128.3, 128.2, 128.1, 128.0, 127.9, 127.8, 127.7, 127.6, 126.7, 85.6, 80.4, 78.5, 75.8, 74.7, 74.6, 73.3, 73.0, 68.2, 32.0, 29.7, 29.6, 29.4, 29.3, 29.1, 28.5, 22.8, 21.2, 14.2.

### <Example 6>

The reaction shown in the following scheme was carried out to produce compound 7 from compound 6.

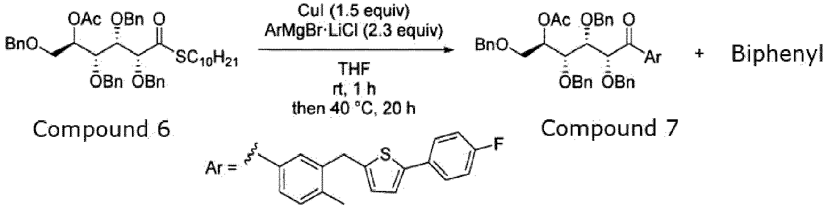

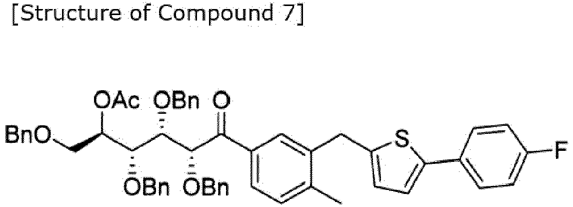

### Preparation of a solution of 0.25 M ArMgBr·LiCl in THF

To LiCl (32.0 mg, 0.755 mmol, 1.01 equivalents) dried *in vacuo* at 100°C for one hour, THF (1.3 mL) was added to obtain a solution. To the solution, while it was cooled at 0°C, a solution of 2.0 M iPrMgCl in THF (0.4 mL, 0.8 mmol, 1.07 equivalents) was added. Note that, "iPr" stands for an isopropyl group. Subsequently, a solution of ArI (2-(5-iodo-2-methylbenzyl)-5-(4-fluorophenyl)thiophene, 306 mg, 0.750 mmol, 1.0 equivalent) in THF (1.3 mL) was slowly added dropwise at 0°C, and thereafter, the reaction mixture was stirred at room temperature for one hour. It was confirmed by TLC that ArI was consumed and ArMgBr·LiCl was produced. ArMgBr·LiCl was used in the following reaction.

### Ketonization reaction

To a suspension of CuI (71.4 mg, 0.375 mmol, 1.5 equivalents) in dry THF (0.5 mL), a solution of 0.25 M ArMgBr·LiCl in THF (2.25 mL, 0.563 mmol, 2.3 equivalents) was added dropwise over 5 minutes and the reaction solution was stirred for 10 minutes. To the reaction solution, a solution of compound 6 (189 mg, 0.250 mmol, 1.0 equivalent) in THF (1.5 mL) was added dropwise over 5 minutes. The reaction solution was stirred at 40°C for 20 hours. The reaction was monitored by TLC (ethyl acetate/n-hexane = 1:5). After completion of the reaction, the reaction was quenched with a 1 M aqueous HCl solution (1 mL). To the reaction solution quenched, ethyl acetate (10 mL) was added. The resultant organic layer was washed with a 1 M aqueous HCl solution (5 mL × 3) and brine (5 mL × 1), and thereafter dried over Na₂SO₄. The mixture was purified by silica column chromatography (ethyl acetate/n-hexane = 1:20 to 1:5) to obtain the corresponding ketone compound (compound 7) in a yield of 76% (164 mg, yellow oil) and the thioester compound (compound 6) at a recovery rate of 19% (35.2 mg, yellow oil).

Physical properties of the ketone compound (compound 7) were as follows.
¹H NMR (400 MHz, CDCl₃, 30°C) δ 7.89 (d, J = 1.3 Hz, 1H), 7.76 (dd, J = 7.9, 1.7 Hz, 1H), 7.38-7.27 (m, 9H), 7.24-7.11 (m, 11H), 7.07-7.02 (m, 3H), 7.00-6.94 (m, 3H), 6.54 (d, J = 3.6 Hz, 1H), 5.27 (q, J = 4.7 Hz, 1H), 4.88 (d, J = 4.6 Hz, 2H), 4.70-4.35 (m, 9H), 4.21 (dd, J = 6.5, 4.7 Hz, 1H), 4.05-4.02 (m, 3H), 3.85 (dd, J = 10.4, 5.0 Hz, 1H), 3.62 (dd, J = 10.4, 5.6 Hz, 1H), 2.33 (s, 3H), 1.96 (s, 3H).
¹³C {¹H} NMR (100 MHz, CDCl₃, 30°C) δ 198.5, 170.1, 142.7, 142.6, 138.6, 138.1, 138.0, 137.4, 134.6, 130.7, 130.3, 128.8, 128.6, 128.43, 128.4, 128.3, 128.2, 128.0, 127.9, 127.8, 127.6, 127.3, 127.2, 126.3, 122.9, 115.9, 115.7, 83.0, 80.7, 79.5, 75.5, 74.7, 73.3, 73.2, 72.8, 68.1, 34.2, 21.2, 19.9.

### <Example 7>

The reaction shown in the following scheme was carried out to produce compound 7 from compound 8.

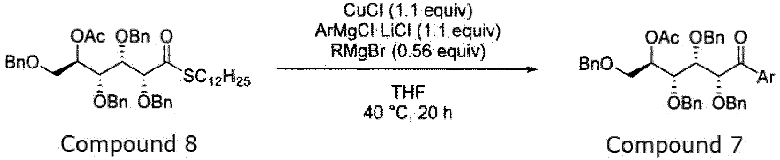

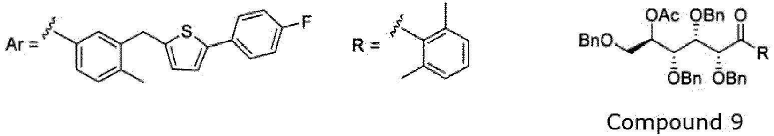

First of all, to a solution of 0.25 M ArMgBr·LiCl in THF was prepared in the same manner as in Example 6. To a suspension of CuCl (27.2 mg, 0.275 mmol, 1.1 equivalents) in dry THF (1.00 mL), a solution of 0.25 M ArMgCl·LiCl in THF (1.10 mL, 0.275 mmol, 1.1 equivalents) was added dropwise over 5 minutes and the mixture was stirred for 10 minutes to obtain a suspension. To the suspension, a solution of 1.0 M RMgBr (2,6-dimethylphenylmagnesium bromide) (0.140 mmol, 0.56 equivalents) in THF (0.140 mL) was added. The mixture was stirred for further 10 minutes to obtain an organic copper reagent.

To the organic copper reagent, a solution of compound 8 (196 mg, 0.250 mmol, 1.0 equivalent) in THF (2.00 mL) was added dropwise over 5 minutes. The mixture was stirred at 40°C for 20 hours. After completion of the reaction, the reaction was quenched with a 1 M aqueous HCl solution (1 mL). To the reaction solution quenched, ethyl acetate (10 mL) was added. The resultant organic layer was washed with a 1 M aqueous HCl solution (5 mL × 3) and brine (5 mL × 1), and thereafter dried over Na₂SO₄. The organic layer dried was purified by silica column chromatography (ethyl acetate:n-hexane = 1:20 to 1:5). As a result, the yield of the ketone derivative (compound 7) was 66% (143 mg) and the yield of compound 8 was 32% (62.6 mg). Note that, fractionation was carried out by silica column chromatography as much as possible but compound 9 that may possibly be produced as a by-product was not confirmed. Similarly on an NMR spectrum, a signal conceivably from compound 9 was not observed.

### <Example 8>

The same operation as in Example 7 was repeated except that RMgBr was not used and the amount of CuCl used was changed to 0.75 equivalents. As a result, the yield of the ketone derivative (compound 7) was 44%.

### <Example 9>

The reaction shown in the following scheme was carried out to produce compound 7 from compound 8.

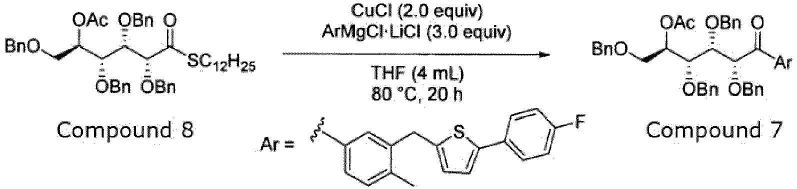

### Preparation of a solution of 0.5 M ArMqBr·LiCl in THF

To LiCl (42.4 mg, 1.00 mmol) dried *in vacuo* at 100°C for one hour, THF (0.500 mL) was added to prepare a solution. To the solution, while it was cooling at 0°C, a solution of 2.0 M iPrMgCl in THF (0.500 mL, 1.00 mmol) was added. Subsequently, a solution of ArI (2-(5-iodo-2-methylbenzyl)-5-(4-fluorophenyl)thiophene, 408 mg, 1.00 mmol) in THF (1.00 mL) was slowly added dropwise at 0°C, and thereafter, the mixture was stirred at room temperature for one hour.

### Ketonization reaction

To a suspension of CuCl (49.5 mg, 0.500 mmol, 2.0 equivalents) in dry THF (1.00 mL), a solution of 0.5 M ArMgBr·LiCl in THF (1.5 mL, 0.750 mmol, 3.0 equivalents) was added dropwise over 5 minutes and the reaction solution was stirred for 10 minutes. To the solution, a solution of compound 8 (196 mg, 0.250 mmol, 1.0 equivalent) in THF (1.5 mL) was added dropwise over 5 minutes. Thereafter the reaction solution was stirred at 80°C for 20 hours. After completion of the reaction, the reaction was quenched with a 1 M aqueous HCl solution (1 mL). To the reaction solution quenched, ethyl acetate (10 mL) was added. The resultant organic layer was washed with a 1 M aqueous HCl solution (5 mL × 3) and brine (5 mL × 1), and thereafter dried over Na₂SO₄. The mixture was purified by silica column chromatography (ethyl acetate/n-hexane = 1:20 to 1:5) to obtain the corresponding ketone compound (compound 7) in a yield of 50% (109 mg) and the thioester compound (compound 8) at a recovery rate of 8% (15.4 mg).

### <Example 10>

The reaction shown in the following scheme was carried out to produce compound 7 from compound 8.

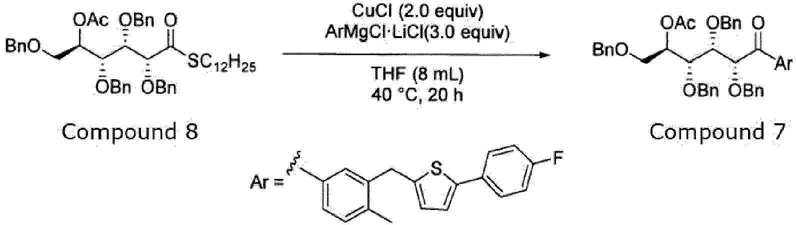

First of all, a solution of 0.25 M ArMgBr·LiCl in THF was prepared in the same manner as in Example 6. To a suspension of CuCl (49.5 mg, 0.500 mmol, 2.0 equivalents) in dry THF (2.00 mL), a solution of 0.25 M ArMgCl·LiCl in THF (3.00 mL, 0.750 mmol, 3.0 equivalents) was added dropwise over 5 minutes and the mixture was stirred for 10 minutes. To the solution, a solution of compound 8 (196 mg, 0.250 mmol, 1.0 equivalent) in THF (3.00 mL) was added dropwise over 5 minutes. Thereafter the solution was stirred at 40°C for 20 hours. After completion of the reaction, the reaction was quenched with a 1 M aqueous HCl solution (1 mL). To the reaction solution quenched, ethyl acetate (10 mL) was added. The resultant organic layer was washed with a 1 M aqueous HCl solution (5 mL × 3) and brine (5 mL × 1), and thereafter dried over Na₂SO₄. The organic layer dried was purified by silica column chromatography (ethyl acetate:n-hexane = 1:20 to 1:5). As a result, the yield of the ketone derivative (compound 7) was 88% (190 mg) and the yield of compound 8 was 6% (11.3 mg).

### <Example 11>

The reaction shown in the following scheme was carried out to produce compound 7 from compound 8.

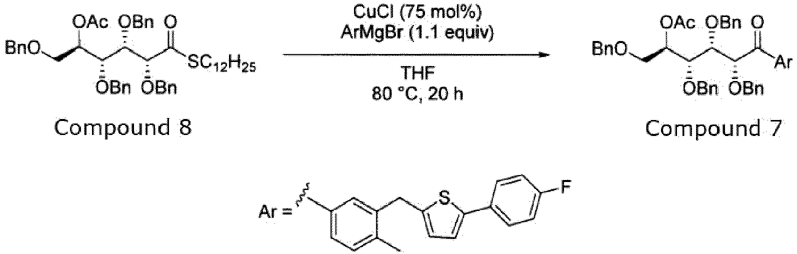

### Preparation of a solution of 0.25 M ArMgBr in THF

To Mg (18.2 mg, 0.750 mmol, 2.0 equivalents), THF (0.500 mL) and 1,2-dibromoethane (0.05 mL) were added to activate Mg. Thereafter a solution of ArBr (2-(5-bromo-2-methylbenzyl)-5-(4-fluorophenyl)thiophene, 135 mg, 0.375 mmol, 1.00 equivalent) in THF (1.00 mL) was slowly added dropwise with attention to heat generation. The mixture was stirred at room temperature for 3 hours.

### Ketonization reaction

To a suspension of CuCl (18.6 mg, 0.188 mmol, 0.75 equivalents) in dry THF (1.00 mL), a solution of 0.25 M ArMgBr in THF (1.10 mL, 0.275 mmol, 1.1 equivalents) was added dropwise over 5 minutes and the mixture was stirred for 10 minutes. To the solution, a solution of compound 8 (196 mg, 0.250 mmol, 1.0 equivalent) in THF (2.00 mL) was added dropwise over 5 minutes. Thereafter the mixture was stirred at 80°C for 20 hours. After completion of the reaction, the reaction was quenched with a 1 M aqueous HCl solution (1 mL). To the reaction solution quenched, ethyl acetate (10 mL) was added. The resultant organic layer was washed with a 1 M aqueous HCl solution (5 mL × 3) and brine (5 mL × 1), and thereafter dried over Na₂SO₄. The mixture was purified by silica column chromatography (ethyl acetate/n-hexane = 1:20 to 1:5) to obtain the corresponding ketone compound (compound 7) in a yield of 42% (90.2 mg) and the thioester compound (compound 8) at a recovery rate of 51% (100 mg).

### <Example 12>

### Production of a thioester derivative

The reaction shown in the following scheme was carried out to produce a thioester derivative (II-iia) from the following lactone derivative (Ia) via a hydroxyl group-containing derivative (II-ia).

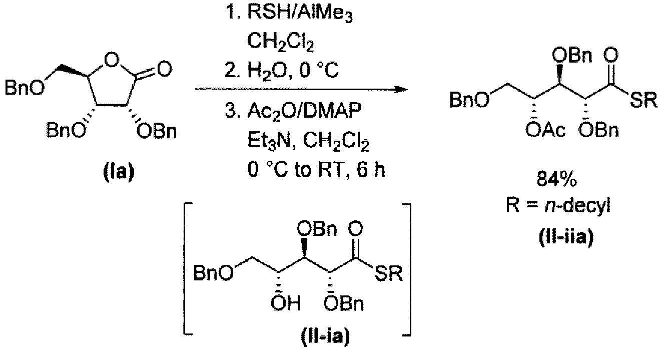

To 20 mL of anhydrous dichloromethane, 0.89 g of 1-decanethiol (5.1 mmol) was added to prepare a 1-decanethiol solution. Trimethylaluminum was dissolved in hexane to prepare a 2 M trimethylaluminum solution. A lactone derivative (Ia) (2.09 g (5 mmol)) was dissolved in 10 mL of anhydrous dichloromethane to prepare a lactone derivative (Ia) solution.

To the 1-decanethiol solution cooled to 0°C, 2.5 mL of a solution of a 2 M trimethylaluminum (trimethylaluminum: 5 mmol) was added dropwise over 10 minutes. The solution was stirred for 20 minutes to obtain a mixed solution. To the mixed solution, the lactone derivative (Ia) solution was slowly added over 20 minutes. The mixture was stirred for 2 hours to obtain a reaction solution. To the reaction solution, 30 mL of dichloromethane was added, and thereafter, the reaction solution was slowly poured in a 500 mL beaker containing 20 mL of ice-cold water. The reaction solution in the beaker was stirred and 40 mL of 1 N hydrochloric acid was slowly added to this, and the reaction solution was allowed to quickly separate into an organic layer and an aqueous layer. After the organic layer was extracted, 30 mL of ice-cold dichloromethane was added to the aqueous layer to separate an organic layer and an aqueous layer. The organic layer was extracted. This operation was repeated further twice. All organic layers were combined to obtain a total organic layer. The total organic layer was washed with water and brine in this order and then dried over sodium sulfate to obtain a residue.

It was confirmed by nuclear magnetic resonance (NMR) spectroscopic analysis that the residue contains a hydroxyl group-containing compound represented by formula (II-ia).

Subsequently, the 3 g of residue (5 mmol) was dissolved in 30 mL of anhydrous dichloromethane to prepare a solution of a hydroxyl group-containing compound (II-ia). To the solution of a hydroxyl group-containing compound (II-ia) cooled up to 0°C in an argon atmosphere, 1.5 mL of acetic anhydride (15.9 mmol) was added and thereafter 13 mg (2 mol%) of 4-dimethylaminopyridine (DMAP) was added. The solution was stirred for 5 minutes. To the solution stirred, 2.2 mL of triethylamine (15 mmol) was added and the mixture was stirred in an argon atmosphere at room temperature for 6 hours to obtain a reaction solution. To the reaction solution, 30 mL of water was added to terminate the reaction. The reaction solution was separated into an organic layer and an aqueous layer. After the organic layer was extracted, 30 mL of dichloromethane was added to the aqueous layer. The solution obtained was separated into an organic layer and an aqueous layer. The organic layer was extracted. This operation was repeated further twice. All organic layers were combined to obtain a total organic layer. The total organic layer was washed with 30 mL of water and 30 mL of brine in this order and then dried over sodium sulfate to obtain a residue. The residue was purified by silica gel column chromatography to obtain the thioester derivative (II-iia) as a transparent liquid. In the silica gel column chromatography, a mixed solvent of ethyl acetate and hexane was used. Volume ratio of ethyl acetate: hexane in the mixed solvent was controlled to be 1:20 to 2:20.

The amount of the thioester derivative (II-iia) was 2.67 g and the yield thereof from the lactone derivative (I) was 84%. NMR spectroscopic analysis results of the thioester derivative (II-iia) were as follows.

¹H NMR (400 MHz, CDCl₃) δ = 7.38-7.18 (m, 15H), 5.31 (dt, J = 6.6, 3.9 Hz, 1H), 4.77 (d, J = 11.7 Hz, 1H), 4.65-4.56 (m, 2H), 4.53 (d, J = 4.4 Hz, 1H), 4.50 (d, J = 5.2 Hz, 1H), 4.40 (d, J = 12.1 Hz, 1H), 4.24-4.16 (m, 2H), 3.70 (d, J = 3.9 Hz, 2H), 2.94-2.79 (m, 2H), 1.98 (s, 3H), 1.61-1.51 (m, 2H), 1.38-1.19 (m, 14H), 0.88 (t, J = 6.8 Hz, 3H).
¹³C NMR (101 MHz, CDCl₃) δ = 200.66, 169.86, 138.27, 137.80, 137.14, 128.50, 128.45, 128.41, 128.16, 128.08, 128.04, 127.84, 127.84, 127.71, 83.97, 79.29, 73.92, 73.55, 73.22, 71.42, 68.43, 32.02, 29.67, 29.62, 29.50, 29.43, 29.27, 29.16, 28.45, 22.81, 21.34, 14.23.
HRMS: [M+H]⁺ C₃₈H₅₁O₆S
Calculated: 635.3406, Found: 635.3403.

### Ketonization reaction

The reaction shown in the following scheme was carried out to produce a compound (9) from a thioester derivative (II-iia).

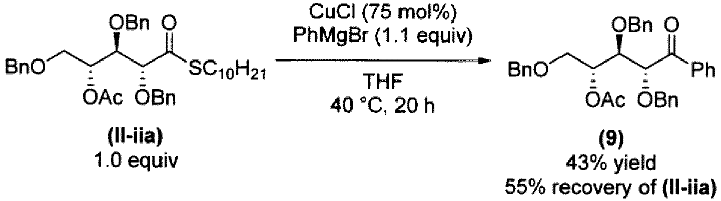

To a suspension of CuCl (18.6 mg, 0.188 mmol, 0.75 equivalents) in dry THF (3.00 mL), a solution of 0.85 M PhMgBr in THF (0.330 mL, 0.281 mmol, 1.1 equivalents) was added dropwise over 5 minutes and the reaction mixture was stirred for 10 minutes. To the solution, a solution of a thioester derivative (II-iia) (166 mg, 0.261 mmol, 1.0 equivalent) in THF (2.00 mL) was added dropwise over 5 minutes and thereafter the solution was stirred at 40°C for 20 hours. After completion of the reaction, the reaction was quenched with a 1 M aqueous HCl solution (1 mL). To the reaction solution quenched, ethyl acetate (10 mL) was added. The resultant organic layer was washed with a 1 M aqueous HCl solution (5 mL × 3) and brine (5 mL × 1), and thereafter dried over Na₂SO₄. The mixture was purified by silica column chromatography (ethyl acetate/n-hexane = 1:20 to 1:5) to obtain the corresponding ketone compound (compound (9)) in a yield of 43% (60.4 mg) and the thioester derivative (II-iia) at a recovery rate of 55% (91.0 mg).

The physical properties of the ketone compound (compound (9)) were as follows.
¹H NMR (400 MHz, CDCl₃, 30°C) δ 7.87 (d, J = 7.2 Hz, 2H), 7.52 (t, J = 7.4 Hz, 1H), 7.38-7.15 (m, 15H), 6.99-6.97 (m, 2H), 5.49 (q, J = 4.8 Hz, 1H), 4.87 (d, J = 6.7 Hz, 1H), 4.64 (d, J = 11.6 Hz, 1H), 4.54-4.34 (m, 5H), 4.20 (dd, J = 6.6, 4.6 Hz, 1H), 3.74-3.73 (m, 2H), 1.96 (s, 3H).

### <Example 13>

The reaction shown in the following scheme was carried out to produce compound 7 from compound 8.

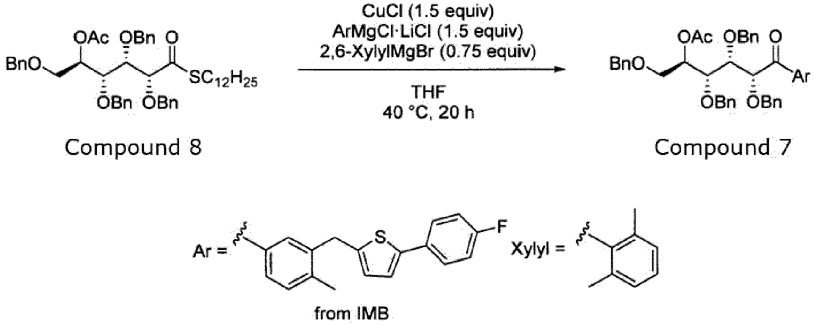

### Preparation of a solution 0.25 M ArMgBr·LiCl in THF

To LiCl (21.2 mg, 0.500 mmol, 1.00 equivalent) dried *in vacuo* at 100°C for one hour, THF (0.750 mL) was added to obtain a solution. Thereafter, to the solution, while it was cooled at 0°C, a solution of 2.0 M iPrMgCl in THF (0.250 mL, 0.500 mmol, 1.00 equivalent) was added. Note that, "iPr" stands for an isopropyl group. Subsequently, a solution of ArI (2-(5-iodo-2-methylbenzyl)-5-(4-fluorophenyl)thiophene (204 mg, 0.500 mmol, 1.00 equivalent) in THF (1.00 mL) was slowly added dropwise at 0°C, and thereafter, the reaction mixture was stirred at room temperature for one hour. It was confirmed by TLC that ArI was consumed and ArMgBr·LiCl was produced. ArMgBr·LiCl was used in the following reaction.

### Ketonization reaction

To a suspension of CuCl (37.1 mg, 0.375 mmol, 1.5 equivalents) in dry THF (1.50 mL), a solution of 0.25 M ArMgBr·LiCl in THF (1.50 mL, 0.375 mmol, 1.5 equivalents) was added dropwise over 5 minutes and the reaction mixture was stirred for 10 minutes to obtain a suspension. To the suspension, a solution of 1.0 M RMgBr (2,6-dimethylphenylmagnesium bromide) (0.188 mmol, 0.75 equivalents) in THF (0.188 mL) was added and the mixture was stirred for further 10 minutes to obtain an organic copper reagent.

To the organic copper reagent, a solution of compound 8 (196 mg, 0.250 mmol, 1.0 equivalent) in THF (1.00 mL) was added dropwise over 5 minutes, and thereafter, the reaction solution was stirred at 40°C for 20 hours. After completion of the reaction, the reaction was quenched with a 1 M aqueous HCl solution (1 mL). To the reaction solution quenched, ethyl acetate (10 mL) was added. The resultant organic layer was washed with a 1 M aqueous HCl solution (5 mL × 3) and brine (5 mL × 1), and thereafter dried over Na₂SO₄. The organic layer dried was purified by silica column chromatography (ethyl acetate:n-hexane = 1:20 to 1:5). As a result, the yield of the ketone derivative (compound 7) was 92% (198 mg) and the yield of compound 8 was 8% (16.1 mg).

### <Example 14>

The same operation as in Example 13 was repeated except that the amount of CuCl used was changed to 2.0 equivalents, the amount of ArMgBr·LiCl used was changed to 2.0 equivalents and the amount of RMgBr used was changed to 1.0 equivalent. As a result, the yield of a ketone derivative (compound 7) was 91% (196 mg) and the yield of compound 8 was 4% (8.6 mg).

### <Example 15>

The reaction shown in the following scheme was carried out to produce compound 7 from compound 8.

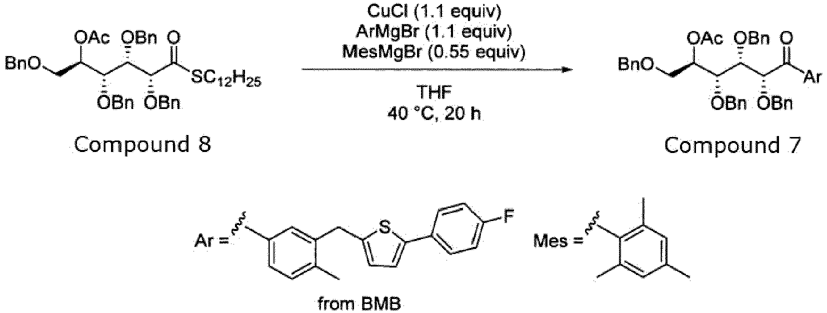

### Preparation of a solution of 0.25 M ArMqBr in THF

To magnesium (24.3 mg, 1.00 mmol, 2.00 equivalents), THF (1.00 mL) and 1,2-dibromoethane (0.05 mL) were added to activate magnesium. Thereafter, to this solution, a solution of BMB (2-(5-bromo-2-methylbenzyl)-5-(4-fluorophenyl)thiophene: 181 mg, 0.500 mmol, 1.00 equivalent) in THF (1.00 mL) was slowly added dropwise. After that, the solution was stirred at 80°C for 3 hours to prepare a solution of 0.25 M ArMgBr in THF.

### Preparation of a solution of 0.25 M MesMgBr in THF

To magnesium (24.3 mg, 1.00 mmol, 2.00 equivalents), THF (1.00 mL) and 1,2-dibromoethane (0.05 mL) were added to activate magnesium. Thereafter, to this solution, a solution of 2-bromomesitylene (99.5 mg, 0.500 mmol, 1.00 equivalent) in THF (1.00 mL) was slowly added dropwise with attention to heat generation. After that, the solution was stirred at room temperature for 3 hours to prepare a solution of 0.25 M MesMgBr in THF.

### Ketonization reaction

To a suspension of CuCl (27.2 mg, 0.275 mmol, 1.1 equivalents) in THF (1.50 mL), a solution of 0.25 M ArMgCl in THF (1.10 mL, 0.275 mmol, 1.1 equivalents) was added dropwise over 5 minutes and the mixture was stirred for 10 minutes to obtain a suspension. To the suspension, a solution of 0.25 M MesMgBr (0.138 mmol, 0.55 equivalents) in THF (0.550 mL) was added. The suspension was stirred for further 10 minutes to obtain an organic copper reagent.

To the organic copper reagent, a solution of compound 8 (196 mg, 0.250 mmol, 1.0 equivalent) in THF (1.00 mL) was added dropwise over 5 minutes, and thereafter, the solution was stirred at 40°C for 20 hours. After completion of the reaction, the reaction was quenched with a 1 M aqueous HCl solution (1 mL). To the reaction solution quenched, ethyl acetate (10 mL) was added. The resultant organic layer was washed with a 1 M aqueous HCl solution (5 mL × 3) and brine (5 mL × 1), and thereafter dried over Na₂SO₄. The organic layer dried was purified by silica column chromatography (ethyl acetate:n-hexane = 1:20 to 1:5). As a result, the yield of the ketone derivative (compound 7) was 56% (120 mg), the yield of compound 8 was 43% (85.0 mg).

### <Example 16A>

The reaction shown in the following scheme was carried out to obtain compound 10 from compound 8.

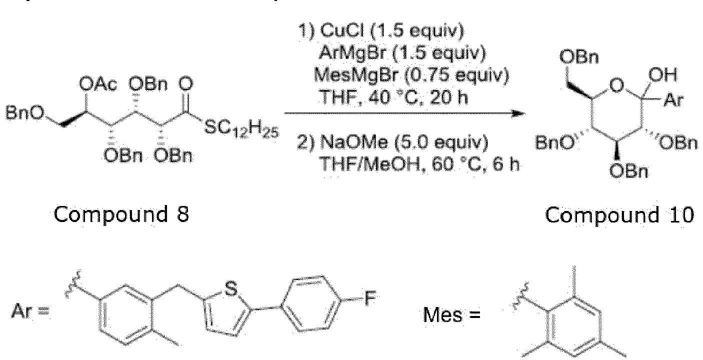

### Preparation of a solution of 0.25 M ArMgBr in THF

To magnesium (48.6 mg, 2.00 mmol, 2.0 equivalents), THF (2.00 mL) and 1,2-dibromoethane (0.05 mL) were added to activate magnesium. Thereafter, to this solution, a solution of BMB (2-(5-bromo-2-methylbenzyl)-5-(4-fluorophenyl)thiophene, 361 mg, 1.00 mmol, 1.00 equivalent) in THF (2.00 mL) was slowly added dropwise. After completion of addition, the mixture was stirred at 80°C for 3 hours.

### Preparation of a solution of 0.25 M MesMqBr in THF

To magnesium (48.6 mg, 2.00 mmol, 2.0 equivalents), THF (2.00 mL) and 1,2-dibromoethane (0.05 mL) were added to activate magnesium. Thereafter, to this solution, a solution of 2-bromomesitylene (200 mg, 1.00 mmol, 1.00 equivalent) in THF (2.00 mL) was slowly added dropwise with attention to heat generation. After completion of addition, the mixture was stirred at 80°C for 3 hours.

### Preparation of copper reagent

To a suspension of CuCl (37.1 mg, 0.375 mmol, 1.5 equivalents) in THF (1.25 mL), a solution of 0.25 M ArMgBr in THF (1.50 mL, 0.375 mmol, 1.5 equivalents) was added dropwise over 5 minutes and the mixture was stirred for 10 minutes. To the solution, a solution of 0.25 M 2-mesitylmagnesium bromide in THF (0.750 mL, 0.188 mmol, 0.75 equivalents) was added and the mixture was stirred for further 10 minutes. The whole amount of the suspension of a copper reagent obtained in THF was used for reaction.

Compound 8 was dissolved in THF (1.50 mL) and the THF solution was added dropwise to the suspension of a copper reagent prepared in THF at room temperature over 5 minutes and the reaction mixture was stirred at 40°C for 20 hours. After cooling to room temperature, a solution of sodium methoxide (67.5 mg, 1.25 mmol, 5.0 equivalents) in methanol (5.00 mL) was added to the reaction mixture and stirred at 60°C for 6 hours. The reaction was terminated with a 1 M aqueous HCl solution (1 mL) and ethyl acetate (10 mL) was added to the reaction mixture. The resultant organic layer was washed with a 1 M aqueous HCl solution (5 mL × 3) and brine (5 mL x 1) and thereafter dried over Na₂SO₄. The mixture was purified by silica gel column chromatography (ethyl acetate: n-hexane = 1: 10 to 1:3) to obtain compound 10 in a yield of 69% (141 mg, yellow oil).

### <Example 16B>

The reaction shown in the following scheme was carried out to produce compound 10 from compound 8.

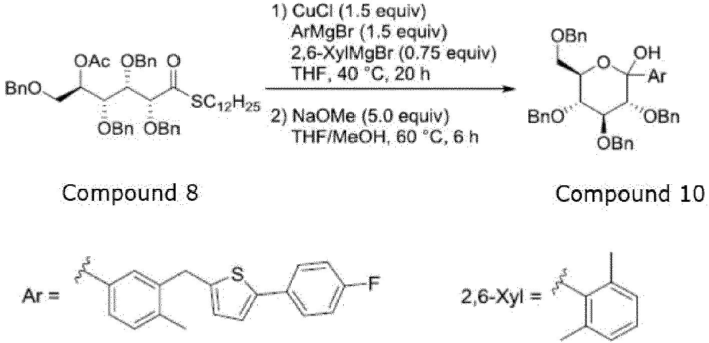

### Preparation of a solution of 0.25 M ArMgBr in THF

To magnesium (48.6 mg, 2.00 mmol, 2.0 equivalents), THF (2.00 mL) and 1,2-dibromoethane (0.05 mL) were added to activate magnesium. Thereafter, to this solution, a solution of BMB (2-(5-bromo-2-methylbenzyl)-5-(4-fluorophenyl)thiophene, 361 mg, 1.00 mmol, 1.00 equivalent) in THF (2.00 mL) was slowly added dropwise. After completion of addition, the solution was stirred at 80°C for 3 hours.

### Preparation of a copper reagent

To a suspension of CuCl (37.1 mg, 0.375 mmol, 1.5 equivalents) in THF (1.81 mL), a solution of 0.25 M ArMgBr in THF (1.50 mL, 0.375 mmol, 1.5 equivalents) was added dropwise over 5 minutes and the reaction mixture was stirred for 10 minutes. To the solution, a solution of 1 M 2,6-xylylmagnesium bromide in THF (Aldrich: 425508-100ML, 0.188 mL, 0.188 mmol, 0.75 equivalents) was added and the mixture was stirred for further 10 minutes. The whole amount of the suspension of a copper reagent obtained in THF was used for reaction.

### Synthesis of lactol

Compound 8 was dissolved in THF (1.50 mL) and added dropwise to the suspension of a copper reagent prepared in THF at room temperature for 5 minutes, and thereafter the reaction mixture was stirred at 40°C for 20 hours. After the reaction mixture was allowed to cool up to room temperature, a solution of sodium methoxide (67.5 mg, 1.25 mmol, 5.0 equivalents) in methanol (5.00 mL) was added to the reaction mixture and the reaction mixture was stirred at 60°C for 6 hours. The reaction was terminated with a 1 M aqueous HCl solution (1 mL), and ethyl acetate (10 mL) was added to the reaction solution. The resultant organic layer was washed with a 1 M aqueous HCl solution (5 mL × 3) and brine (5 mL × 1), and thereafter dried over Na₂SO₄. The mixture was purified by silica gel column chromatography (ethyl acetate: n-hexane = 1:10 to 1:3) to obtain compound 10 in a yield of 77% (157 mg, yellow oil).

¹H NMR data of compound 10 obtained in Examples 16A and B were as follows:
¹H NMR (400 MHz, CDCl₃, 30°C) δ 7.52 (d, J = 1.8 Hz, 1H), 7.45 (dd, J = 7.8, 1.9 Hz, 1H), 7.38-7.28 (m, 10H), 7.26-7.15 (m, 11H), 7.00-6.94 (m, 5H), 6.61 (d, J = 3.6 Hz, 1H), 4.89-4.86 (m, 3H), 4.67 (d, J = 8.4 Hz, 1H), 4.64 (d, J = 9.8 Hz, 1H), 4.54 (d, J = 12.4 Hz, 1H), 4.38 (d, J = 10.6 Hz, 1H), 4.18-4.03 (m, 4H), 3.94 (d, J = 10.6 Hz, 1H), 3.88-3.82 (m, 2H), 3.72 (dd, J = 11.1, 1.8 Hz, 1H), 3.60 (dd, J = 9.3, 0.8 Hz, 1H), 3.06 (d, J = 0.9 Hz, 1H), 2.33 (s, 3H).

### <Example 17>

The reaction shown in the following scheme was carried out to produce compound 12a from compound 11a. In the same manner, compound 12b to 12k were produced from compound 11b to 11k, respectively.

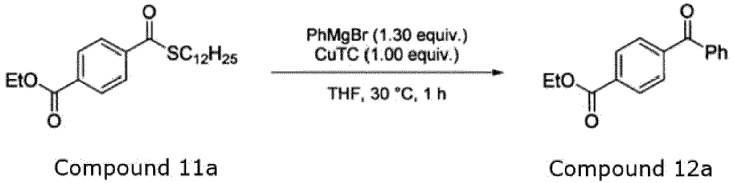

| | | |
|---|---|---|
| 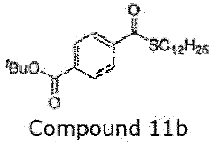 | 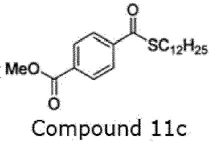 | 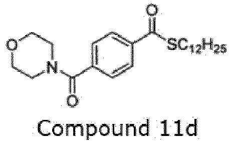 |
| 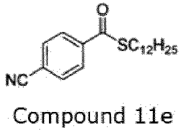 | 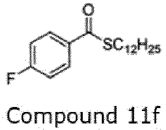 | 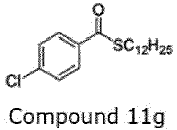 |
| 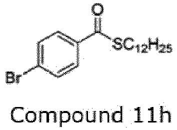 | 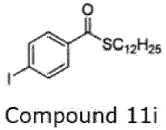 | 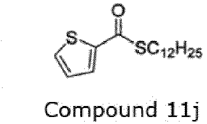 |
| 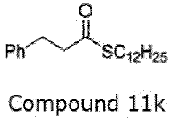 | | |

| | | |
|---|---|---|
| 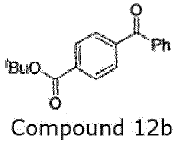 | 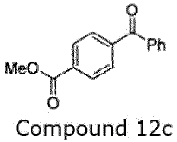 | 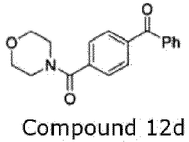 |
| 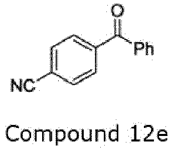 | 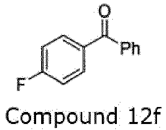 | 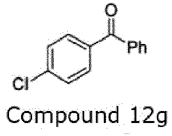 |
| 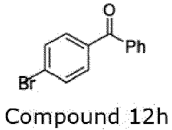 | 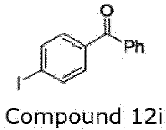 | 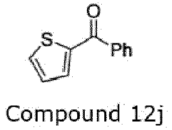 |
| 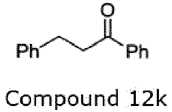 | | |

### Production of thioesters (compounds 11a to 11k)

The reaction shown in the following scheme was carried out to produce desired thioesters.

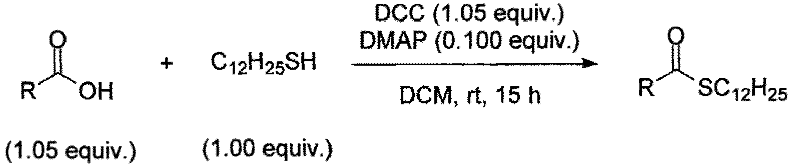

To a suspension containing carboxylic acid (1.05 equivalents) corresponding to a desired thioester, thiol (1.00 equivalent) and 4-dimethylaminopyridine (DMAP) (0.100 equivalent) in dichloromethane (DCM), N,N'-dicyclohexylcarbodiimide (DCC) (1.05 equivalents) was added at 0°C. The reaction mixture was stirred at 0°C for 30 minutes and thereafter stirred at room temperature (rt) for 15 hours. After completion of the reaction, a precipitate was removed by Celite filtration and the filtrate was washed with 1 M aqueous HCl solution, saturated sodium bicarbonate water and saturated brine. After the solvent was distilled away, a crude product was purified by silica gel column chromatography (ethyl acetate/n-hexane = 1:30 -> 1:2) to obtain the desired thioester.

Compound 11a was obtained as a white solid (3.04 g, 8.03 mmol, yield 72%). Analysis results of compound 11a were as follows.
¹H NMR (400 MHz, CDCl₃, 30°C) δ 8.11 (d, J = 8.6 Hz, 2H, ArH), 8.01 (d, J = 8.6 Hz, 2H, ArH), 4.41 (q, J = 7.1 Hz, 2H, OCH₂CH₃), 3.09 (t, J = 7.3 Hz, 2H, SCH₂), 1.69 (quint, J = 7.1 Hz, 2H, SCH₂CH₂), 1.43-1.26 (m, 21H, SCH₂CH₂(CH₂)₉CH₃+OCH₂CH₃), 0.88 (t, J = 6.6 Hz, 3H, S(CH₂)₁₁CH₃).
¹³C {¹H} NMR (100 MHz, CDCl₃, 30°C) δ 191.7, 165.8, 140.6, 134.5, 129.9, 127.2, 61.6, 32.0, 29.8, 29.7₄, 29.6₉, 29.6₉, 29.5₅, 29.5, 29.3, 29.1, 22.8, 14.4, 14.2.

Compound 11b was obtained as a white solid (1.47 g, 3.62 mmol, yield 89%). Analysis results of compound 11b were as follows.
¹H NMR (400 MHz, CDCl₃, 30°C) δ 8.05-7.97 (m, 4H, ArH), 3.08 (t, J = 7.2 Hz, 2H, SCH₂), 1.68 (quint, J = 7.5 Hz, 2H, SCH₂CH₂), 1.61 (s, 9H, ^{t}Bu), 1.43 (m, 2H, SCH₂CH₂CH₂), 1.30-1.26 (m, 16H, SCH₂CH₂CH₂(CH₂)₈CH₃), 0.88 (t, J = 6.7 Hz, 3H, S(CH₂)₁₁CH₃).
¹³C {¹H} NMR (100 MHz, CDCl₃, 30°C) δ 191.8, 164.9, 140.3, 136.1, 129.7, 127.5, 127.1, 81.9, 32.0, 29.7₄, 29.6₉, 29.6₀, 29.5₆, 29.4₆, 29.4, 29.3, 29.1, 28.3, 22.8, 14.2.
IR (neat KBr, v/cm⁻¹) 2952, 2915, 2871, 2850, 1728, 1657, 1607, 1542.
HRMS (FAB⁺) m/z C₄₀H₄₉O₃S ([M+H]⁺)
Calculated: 406.2542, Found: 407.2616.
Melting point: 50.8 to 52.0°C.

Compound 11c was obtained as a white solid (6.20 g, 17.0 mmol, yield 85%). Analysis results of compound 11c were as follows.
¹H NMR (400 MHz, CDCl₃, 30°C) δ 8.10 (d, J = 8.0 Hz, 2H, ArH), 8.01 (d, J = 8.0 Hz, 2H, ArH), 3.94 (s, 3H, OCH₃), 3.09 (t, J = 8.0 Hz, 2H, SCH₂), 1.68 (quint, J = 8.0 Hz, 2H, SCH₂CH₂), 1.41-1.43 (m, 2H, SCH₂CH₂CH₂), 1.26-1.30 (m, 16H, SCH₂CH₂CH₂(CH₂)₈CH₃), 0.88 (t, J = 6.0 Hz, 3H, S(CH₂)₁₁CH₃).
¹³C {¹H} NMR (100 MHz, CDCl₃, 30°C) δ 191.7, 166.3, 140.7, 134.2, 129.9, 127.2, 66.2, 56.1, 52.6, 52.2, 32.0, 29.7, 29.7, 29.6, 29.5, 29.5, 29.3, 29.0, 22.8, 14.2.
IR (neat KBr, v/cm⁻¹) 2952, 2917, 2850, 1715, 1656.
HRMS (FAB⁺) m/z C₂₄H₄₉O₃S ([M+H]⁺)
Calculated: 364.2072, Found: 365.2151.
Melting point: 68.3 to 70.7°C.

Compound 11d was obtained as a white solid (1.25 g, 2.98 mmol, yield 92%). Analysis results of compound 11d were as follows.
¹H NMR (400 MHz, CDCl₃, 30°C) δ 8.01 (d, J = 8.2 Hz, 2H, ArH), 7.47 (d, J = 8.2 Hz, 2H, ArH), 3.77-3.40 (m, 8H, morpholine), 3.08 (t, J = 8.0 Hz, 2H, SCH₂), 1.68 (quint, J = 8.0 Hz, 2H, SCH₂CH₂), 1.43-1.40 (m, 2H, SCH₂CH₂CH₂), 1.26 (m, 16H, SCH₂CH₂CH₂(CH₂)₈CH₃), 0.88 (t, J = 6.0 Hz, 3H, S(CH₂)₁₁CH₃).
¹³C {¹H} NMR (100 MHz, CDCl₃, 30°C) δ 191.5, 169.3, 139.9, 138.4, 127.6, 127.4, 66.9, 48.2, 42.7, 32.0, 29.7₃, 29.6₉, 29.6₉, 29.5₇, 29.5, 29.4, 29.3, 29.0, 22.8, 14.2.
IR (neat KBr, v/cm⁻¹) 2951, 2917, 2849, 1661, 1920, 1604.
HRMS (FAB⁺) m/z C₄₉H₄₉NO₃S ([M+H]⁺)
Calculated: 419.2494, Found: 420.2571.
Melting point: 75.9 to 77.1°C.

Compound 11e was obtained as a white solid (0.792 g, 2.39 mmol, yield 80%). Analysis results of compound 11e were as follows.
¹H NMR (400 MHz, CDCl₃, 30°C) δ 8.05 (d, J = 8.5 Hz, 2H, ArH), 7.75 (d, J = 8.4 Hz, 2H, ArH), 3.10 (t, J = 7.3 Hz, 2H, SCH₂), 1.69 (quint, J = 7.1 Hz, 2H, SCH₂CH₂), 1.43-1.41 (m, 2H, SCH₂CH₂CH₂), 1.32-1.26 (m, 16H, SCH₂CH₂CH₂(CH₂)₈CH₃), 0.88 (t, J = 6.6 Hz, 3H, S(CH₂)₁₁CH₃).
¹³C {¹H} NMR (100 MHz, CDCl₃, 30°C) δ 190.9, 140.5, 132.6, 127.8, 118.0, 116.6, 32.0, 29.7₄, 29.6₇, 29.6₄, 29.5s, 29.5, 29.2, 29.0, 22.8, 14.2.
IR (neat KBr, v/cm⁻¹) 2952, 2915, 2870, 2851, 1658, 1623, 1560, 1542.
HRMS (FAB⁺) m/z C₂₄H₄₉NOS ([M+H]⁺)
Calculated: 331.1970, Found: 332.2047.
Melting point: 50.6 to 53.1°C.

Compound 11f was obtained as a white solid (0.887 g, 2.73 mmol, yield 91%). Analysis results of compound 11f were as follows.
¹H NMR (400 MHz, CDCl₃, 30°C) δ 8.02-7.98 (m, 2H, ArH), 7.14-7.10 (m, 2H, ArH), 3.07 (t, J = 7.3 Hz, 2H, SCH₂), 1.67 (quint, J = 7.0 Hz, 2H, SCH₂CH₂), 1.43-1.27 (m, 18H, SCH₂CH₂(CH₂)₉CH₃), 0.89 (t, J = 6.6 Hz, 3H, S(CH₂)₁₁CH₃).
¹³C {¹H} NMR (100 MHz, CDCl₃, 30°C) δ 190.7, 166.0 (d, J_{C-F} = 253 Hz), 133.8, 129.8 (d, J_{C-F} = 10 Hz), 115.7 (d, J_{C-F} = 22 Hz), 32.0, 29.7₆, 29.7₅, 29.7₀, 29.6₇, 29.6, 29.5, 29.3₂, 29.2₇, 29.1, 22.8, 14.2.
¹⁹F {¹H} NMR (376 MHz, CDCl₃, 30°C) δ -105.2.

Compound 11g was obtained as a white solid (0.702 g, 2.06 mmol, yield 69%). Analysis results of compound 11g were as follows.
¹H NMR (400 MHz, CDCl₃, 30°C) δ 7.91 (d, J = 8.4 Hz, 2H, ArH), 7.42 (d, J = 8.4 Hz, 2H, ArH), 3.07 (t, J = 7.4 Hz, 2H, SCH₂), 1.67 (quint, J = 7.2 Hz, 2H, SCH₂CH₂), 1.43-1.27 (m, 18H, SCH₂CH₂(CH₂)₉CH₃), 0.89 (t, J = 6.3 Hz, 3H, S(CH₂)₁₁CH₃).
¹³C {¹H} NMR (100 MHz, CDCl₃, 30°C) δ 191.2, 139.8, 135.9, 129.1, 128.8, 32.2, 29.9₁, 29.8₅, 29.8, 29.6, 29.5, 29.4, 29.2, 23.0, 14.4.

Compound 11h was obtained as a white solid (0.996 g, 2.58 mmol, yield 87%). Analysis results of compound 11h were as follows.
¹H NMR (400 MHz, CDCl₃, 30°C) δ 7.83 (d, J = 8.6 Hz, 2H, ArH), 7.58 (d, J = 8.6 Hz, 2H, ArH), 3.07 (t, J = 7.3 Hz, 2H, SCH₂), 1.67 (quint, J = 7.1 Hz, 2H, SCH₂CH₂), 1.42-1.40 (m, 2H, SCH₂CH₂CH₂), 1.26 (m, 16H, SCH₂CH₂CH₂(CH₂)₈CH₃), 0.88 (t, J = 6.6 Hz, 3H, S(CH₂)₁₁CH₃).
¹³C {¹H} NMR (100 MHz, CDCl₃, 30°C) δ 191.3, 136.2, 132.0, 128.8, 128.3, 32.0, 29.7₄, 29.7₃, 29.6₈, 29.5₉, 29.5₈, 29.5, 29.3₄, 29.2₅, 29.0, 22.8, 14.2.

Compound 11i was obtained as a white solid (1.23 g, 2.84 mmol, yield 95%). Analysis results of compound 11i were as follows.
¹H NMR (400 MHz, CDCl₃, 30°C) δ 7.81 (d, J = 8.5 Hz, 2H, ArH), 7.68 (d, J = 8.6 Hz, 2H, ArH), 3.07 (t, J = 7.3 Hz, 2H, SCH₂), 1.67 (quint, J = 7.0 Hz, 2H, SCH₂CH₂), 1.42-1.40 (m, 2H, SCH₂CH₂CH₂), 1.27 (m, 16H, SCH₂CH₂CH₂(CH₂)₈CH₃), 0.89 (t, J = 6.6 Hz, 3H, S(CH₂)₁₁CH₃).
¹³C {¹H} NMR (100 MHz, CDCl₃, 30°C) δ 191.3, 137.8, 136.6, 128.5, 100.7, 31.8, 29.6, 29.5₄, 29.4₉, 29.4₁, 29.3s, 29.3, 29.1₁, 29.0₆, 28.8, 22.6, 14.0.
IR (neat KBr, v/cm⁻¹) 2952, 2915, 2869, 2847, 1651, 1578, 1559, 1541, 1508.
HRMS (FAB⁺) m/z C₂₄H₄₉OSI ([M+H]⁺) Calculated: 432.0984, Found: 433.1055.
Melting point: 47.6 to 48.6°C.

Compound 11j was obtained as a white solid (0.955 g, yield 51%). Analysis results of compound 11j were as follows.
¹H NMR (400 MHz, CDCl₃, 30°C) δ 7.80 (d, J = 3.8 Hz, 1H, thiophene), 7.60 (d, J = 4.9 Hz, 1H, thiophene), 7.10 (d, J = 4.0 Hz, 1H, thiophene), 3.07 (t, J = 7.3 Hz, 2H, SCH₂), 1.67 (quint, J = 7.0 Hz, 2H, SCH₂CH₂), 1.42-1.40 (m, 2H, SCH₂CH₂CH₂), 1.27 (m, 16H, SCH₂CH₂CH₂(CH₂)₈CH₃), 0.88 (t, J = 6.6 Hz, 3H, S(CH₂)₁₁CH₃).
¹³C {¹H} NMR (100 MHz, CDCl₃, 30°C) δ 184.3, 142.6, 132.4, 130.9, 127.9, 32.0, 29.7₈, 29.7₆, 29.7₄, 29.7₀, 29.6, 29.5, 29.4, 29.3, 29.0, 22.8, 14.2.

Compound 11k was obtained as a colorless liquid (2.55 g, yield 85%). Analysis results of compound 11k were as follows.
¹H NMR (400 MHz, CDCl₃, 30°C) δ 7.30-7.25 (m, 2H, ArH), 7.21-7.17 (m, 3H, ArH), 2.98 (t, J = 7.2 Hz, 2H, SCH₂), 2.89-2.83 (m, 4H, PhCH₂CH₂), 1.55 (quint, J = 7.2 Hz, 2H, SCH₂CH₂), 1.26 (m, 18H, SCH₂CH₂(CH₂)₉CH₃), 0.89 (t, J = 7.0 Hz, 3H, S(CH₂)₁₁CH₃).
¹³C {¹H} NMR (100 MHz, CDCl₃, 30°C) δ 198.8, 140.3, 128.6, 128.4, 126.4, 45.7, 32.1, 31.6, 29.7₈, 29.7₆, 29.7₁, 28.6₇, 29.6, 29.5, 29.3, 29.1, 28.9, 22.8, 14.2.

### Ketonization reaction (production of compounds 12a to 12k)

To a suspension of CuTC (copper (I) thiophene-2-carboxylate, 0.250 mmol, 47.7 mg, 1.0 equivalent) in THF (2 mL), a solution of 0.52 M PhMgBr in THF (0.325 mmol, 0.625 mL, 1.3 equivalents) was added dropwise over 5 minutes, and thereafter, the reaction mixture was stirred at room temperature for 10 minutes. In this stage, an organic copper reagent was produced. The organic copper reagent produced was added dropwise to the solution of thioester (0.250 mmol, 1.0 equivalent) in THF (2 mL) over 5 minutes, and thereafter, the reaction mixture was stirred at 30°C for one hour. After completion of the reaction, the reaction was terminated with a 1 M aqueous HCl solution (1 mL) and the yield was calculated in accordance with the following method.

### [Yield]

Yields were calculated based on a proton integral ratio of an internal standard substance (triphenylmethane) and a product. A sample was prepared as follows: triphenylmethane serving as an internal standard was added; metal salts were removed by short pad silica (ethyl acetate); a solvent was distilled away under reduced pressure, and the resultant product was dissolved in deuterated chloroform.

Compound 12a was obtained as a colorless oil (52.1 mg, 0.205 mmol, yield 82%). The analysis results of compound 12a were as follows.
¹H NMR (400 MHz, CDCl₃, 30°C) δ 8.15 (d, J = 8.2 Hz, 2H, ArH), 7.83 (d, J = 8.2 Hz, 2H, ArH), 7.80 (d, J = 7.5 Hz, 2H, ArH), 7.61 (t, J = 7.3 Hz, 1H, ArH), 7.49 (t, J = 7.6 Hz, 2H, ArH), 4.42 (q, J = 7.1 Hz, 2H, OCH₂), 1.42 (t, J = 7.2 Hz, 3H, OCH₂CH₃).
¹³C {¹H} NMR (100 MHz, CDCl₃, 30°C) δ 196.1, 165.9, 141.4, 137.2, 133.7, 133.0, 130.2, 129.8, 129.6, 128.6, 61.5, 14.4.

Compound 12b was obtained as a colorless oil (56.7 mg, 0.201 mmol, yield 81%). The analysis results of compound 12b were as follows.
¹H NMR (400 MHz, CDCl₃, 30°C) δ 8.09 (d, J = 8.3 Hz, 2H, ArH), 7.81 (d, J = 8.0 Hz, 2H, ArH), 7.79 (d, J = 6.8 Hz, 2H, ArH), 7.61 (t, J = 7.3 Hz, 2H, ArH), 7.49 (t, J = 7.8 Hz, 2H, ArH), 1.62 (s, 9H, O^{t}Bu).
¹³C {¹H} NMR (100 MHz, CDCl₃, 30°C) δ 196.1, 164.9, 140.9, 137.1, 135.2, 132.8, 130.1, 129.6, 129.3, 128.4, 81.8, 28.1.

Compound 12c was obtained as a white solid by recycling HPLC (49.3 mg, purity 93%, 0.190 mmol, yield 76%). The analysis results of compound 12c were as follows.
¹H NMR (400 MHz, CDCl₃, 30°C) δ 8.15 (d, J = 8.4 Hz, 2H, ArH), 8.15 (d, J = 8.0 Hz, 2H, ArH), 8.15 (d, J = 8.0 Hz, 2H, ArH), 7.61 (t, J = 7.4 Hz, 1H, ArH), 7.49 (t, J = 7.4 Hz, 2H, ArH), 3.96 (s, 3H, OMe).
¹³C {¹H} NMR (100 MHz, CDCl₃, 30°C) δ 196.0, 166.3, 141.3, 137.0, 132.9, 130.1, 129.7, 129.5, 128.4, 52.4.

Compound 12d was obtained as a colorless oil (62.3 mg, 0.211 mmol, yield 84%). The analysis results of compound 12d were as follows.
¹H NMR (400 MHz, CDCl₃, 30°C) δ 7.85-7.79 (m, 4H, ArH), 7.63-7.59 (m, 1H, ArH), 7.53-7.47 (m, 4H, ArH), 3.78-3.45 (m, 8H, morpholine).
¹³C {¹H} NMR (100 MHz, CDCl₃, 30°C) δ 196.0, 169.5, 139.1, 139.0, 137.2, 133.0, 130.3, 130.2, 128.6, 127.1, 67.0, 48.1, 42.8.

Compound 12e was obtained as a white solid (41.0 mg, 0.198 mmol, yield 79%). The analysis results of compound 12e were as follows.
¹H NMR (400 MHz, CDCl₃, 30°C) δ 7.88 (d, J = 8.3 Hz, 2H, ArH), 7.79 (m, 4H, ArH), 7.65 (t, J = 7.3 Hz, 2H, ArH), 7.52 (t, J = 7.7 Hz, 2H, ArH).
¹³C {¹H} NMR (100 MHz, CDCl₃, 30°C) δ 195.1, 141.4, 136.5, 133.4, 132.3, 130.4, 130.2, 128.8, 118.1, 115.8.

Compound 12f was obtained as a colorless oil (41.8 mg, 0.209 mmol, yield 84%). The analysis results of compound 12f were as follows.
¹H NMR (400 MHz, CDCl₃, 30°C) δ 7.86-7.83 (m, 2H, ArH), 7.77 (d, J = 7.3 Hz, 2H, ArH), 7.59 (t, J = 7.4 Hz, 1H, ArH), 7.49 (t, J = 7.8 Hz, 2H, ArH), 7.16 (t, J = 8.6 Hz, 2H, ArH).
¹³C {¹H} NMR (100 MHz, CDCl₃, 30°C) δ 195.2, 165.3 (d, J_{C-F} = 252 Hz), 137.5, 133.8 (d, J_{C-F} = 3 Hz), 132.6 (d, J_{C-F} = 9 Hz), 132.4, 129.8, 128.3, 115.4 (d, J_{C-F} = 22 Hz).
¹⁹F {¹H} NMR (376 MHz, CDCl₃, 30°C) δ -106.0.

Compound 12g was obtained as a white solid (49.0 mg, 0.226 mmol, yield 91%). The analysis results of compound 12g were as follows.
¹H NMR (400 MHz, CDCl₃, 30°C) δ 7.78 (d, J = 6.4 Hz, 2H, ArH), 7.76 (d, J = 8.3 Hz, 2H, ArH), 7.60 (t, J = 7.3 Hz, 1H, ArH), 7.49 (t, J = 7.8 Hz, 2H, ArH), 7.46 (d, J = 8.4 Hz, 2H, ArH).
¹³C {¹H} NMR (100 MHz, CDCl₃, 30°C) δ 195.4, 138.9, 137.3, 135.9, 132.6, 131.4, 129.9, 128.6, 128.4.

Compound 12h was obtained as a white solid (57.2 mg, 0.219 mmol, yield 88%). The analysis results of compound 12h were as follows.
¹H NMR (400 MHz, CDCl₃, 30°C) δ 7.77 (d, J = 7.1 Hz, 2H, ArH), 7.68 (d, J = 8.6 Hz, 2H, ArH), 7.63 (d, J = 8.5 Hz, 2H, ArH), 7.59 (d, J = 7.4 Hz, 1H, ArH), 7.49 (t, J = 7.5 Hz, 2H, ArH).
¹³C {¹H} NMR (100 MHz, CDCl₃, 30°C) δ 195.7, 137.4, 136.5, 132.8, 131.8, 131.7, 130.1, 128.5, 127.6.

Compound 12i was obtained as a white solid (59.6 mg, 0.193 mmol, yield 77%). The analysis results of compound 12i were as follows.
¹H NMR (400 MHz, CDCl₃, 30°C) δ 7.85 (d, J = 8.4 Hz, 2H, ArH), 7.77 (d, J = 7.2 Hz, 2H, ArH), 7.60 (t, J = 7.4 Hz, 1H, ArH), 7.53-7.47 (m, 4H, ArH).
¹³C {¹H} NMR (100 MHz, CDCl₃, 30°C) δ 196.0, 137.7, 137.3, 137.1, 132.8, 131.6, 130.1, 128.5, 100.2.

Compound 12j was obtained as a yellow oil (34.3 mg, 0.182 mmol, yield 73%). The analysis results of compound 12j were as follows.
¹H NMR (400 MHz, CDCl₃, 30°C) δ 7.87 (d, J = 7.4 Hz, 2H, ArH), 7.72 (d, J = 5.0 Hz, 1H, thiophene), 7.66 (d, J = 3.8 Hz, 1H, thiophene), 7.59 (t, J = 7.2 Hz, 1H, ArH), 7.50 (t, J = 7.8 Hz, 2H, ArH), 7.16 (t, J = 4.0 Hz, 1H, thiophene).
¹³C {¹H} NMR (100 MHz, CDCl₃, 30°C) δ 188.3, 143.8, 138.3, 134.9, 134.3, 132.4, 129.3, 128.5, 128.1.

Compound 12k was obtained as a white solid (37.8 mg, 0.180 mmol, yield 72%). The analysis results of compound 12k were as follows.
¹H NMR (400 MHz, CDCl₃, 30°C) δ 7.95 (d, J = 7.5 Hz, 2H, ArH), 7.54 (t, J = 7.4 Hz, 2H, ArH), 7.44 (t, J = 7.7 Hz, 2H, ArH), 7.31-7.27 (m, 3H, ArH), 7.21-7.17 (m, 2H, ArH), 3.29 (t, J = 7.7 Hz, 2H, PhCH₂CH₂), 3.07 (t, J = 7.7 Hz, 2H, PhCH₂CH₂).
¹³C {¹H} NMR (100 MHz, CDCl₃, 30°C) δ 199.3, 141.4, 137.0, 133.2, 128.7, 128.6₅, 128.6, 128.5₅, 128.4, 128.2, 126.3, 40.6, 30.3.

### <Example 18>

The reaction shown in the following scheme was optimized.

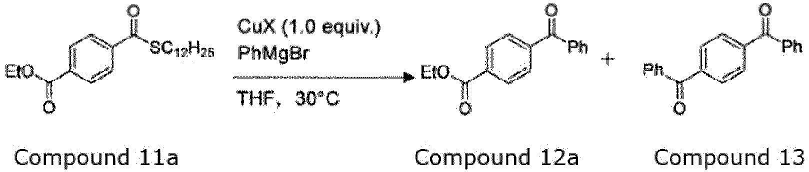

In Example 18a, 1.3 equivalents of PhMgBr was used relative to 1 equivalent of CuCl and the reaction time was set to be 3 hours. More specifically, the amount of CuCl relative to 1 mol of PhMgBr was set to be 0.77 mol. In Example 18b, 1.6 equivalents of PhMgBr was used relative to 1 equivalent of CuCl and the reaction time was set to be 3 hours. More specifically, the amount of CuCl relative to 1 mol of PhMgBr was set to be 0.63 mol. In Example 18c, 1.3 equivalents of PhMgBr was used relative to 1 equivalent of CuTC (copper (I) thiophene-2-carboxylate) and the reaction time was set to be 3 hours. Example 18d, 1.3 equivalents of PhMgBr was used relative to 1 equivalent of CuTC and the reaction time was set to be one hour.

In Examples 18a to 18d, the yields of compounds 12a and 13 were obtained in the same manner as yield described in Example 17. The results are shown in Table 5.

**[Table 5]**

| | Cupper salt (CuX) | Amount of PhMgBr used (equiv.) | |
|---|---|---|---|
| Examples 18a | CuCl | 1.3 | |
| Examples 18b | CuCl | 1.6 | |
| Examples 18c | CuTC | 1.3 | |
| Examples 18d | CuTC | 1.3 | |
| | | | |

| | Yield of Compound 12a (%) | | Yield of Compound 13 (%) |
|---|---|---|---|
| Examples 18a | 78 | | trace amount |
| Examples 18b | 59 | | 11 |
| Examples 18c | 87 | | 9 |
| Examples 18d | 89 | | trace amount |

From the results shown in Table 5, the optimal equivalent ratio of a copper salt to a Grignard reagent (equivalent of copper salt:equivalent of Grignard reagent) was determined as 1:1.3. CuTC exhibited a higher activity than CuCl. Although production of a compound 13 was produced as a by-product, compound 12a was obtained in a higher yield. Compound 12a was obtained in a higher yield by reducing reaction time from 3 hours to one hour.

### <Example 19>

### Preparation of [Ph₂Cu]Mg₂Br₃(thf)₆]

The reaction shown in the following scheme was carried out to prepare [Ph₂Cu][Mg₂Br₃(thf)₆].

2CuCl + 3PhMgBr -> [Ph₂Cu][Mg₂Br₃(thf)₆] + PhCu + MgCl₂

To a solution of CuCl (248 mg, 2.50 mmol, 1.0 equivalent) in THF (5.73 mL), a solution of 0.9 M PhMgBr in THF (4.17 mL, 3.75 mmol, 1.5 equivalents) was added. The mixture was stirred at room temperature for one hour to obtain a yellow-green suspension. To this suspension, toluene (30 mL) was added and the mixture was heated up to 120°C. After the suspension was changed to be a dark green solution, insoluble matter was removed by filtration. Then, the filtrate was slowly cooled up to room temperature. A crystal obtained by crystallization from toluene/THF = 3/1 was washed with toluene (1 mL × 3) and THF (1 mL × 3) to obtain [Ph₂Cu][Mg₂Br₃(thf)₆] as a green brock crystal in a yield of 59% (693 mg). The crystal obtained was suitable for single-crystal X-ray structural analysis (FIG. 1). FIG. 1 shows the molecular structure of [Ph₂Cu][Mg₂Br₃(thf)₆] by 50% thermal ellipsoid. Note that, in FIG. 1, hydrogen atoms are omitted for clarification of the drawing.

### <Example 20>

### Comparative experiment of [Ph₂Cu][Mg₂Br₃(thf)₆] reactivity

(a) 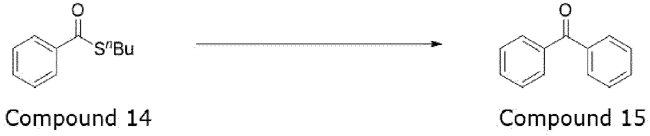
(b) 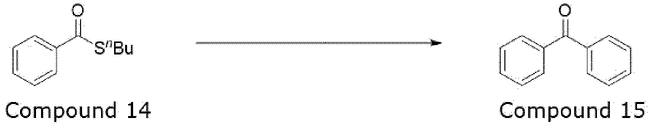
(c) 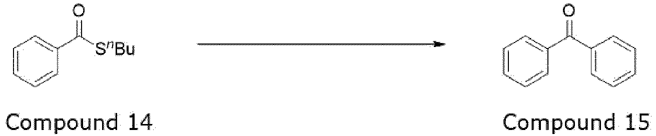
(d) 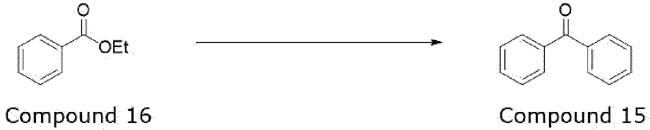
(e)

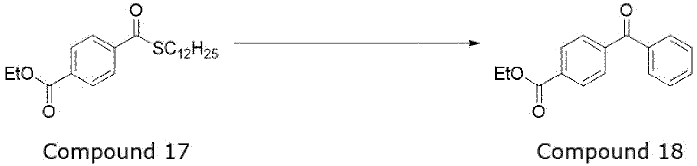

### Preparation of a suspension of 0.5 M CuPh in THF

To a solution of CuBr (1.43 g, 10.0 mmol, 1.00 equivalent) in THF (20.0 mL), a solution of 0.9 M PhMgBr in THF (11.1 mL, 10.0 mmol, 1.00 equivalent) was added dropwise at 0°C. After the mixture was stirred at room temperature for 2 hours, the supernatant was removed by filtration. The residue was washed with THF (10 mL × 3) and dried *in vacuo.* THF (20 mL) was added to obtain a suspension of 0.5 M CuPh in THF.

Operations according to Schemes (a) to (d) were carried out in J-young tubes. Operation according to scheme (e) was carried out in 20 mL J-young Schlenk tube.

### Scheme (a)

To a solution of [Ph₂Cu][Mg₂Br₃(thf)₆] (11.7 mg, 12.5 µmol, 0.500 equivalents or 23.5 mg, 25.0 µmol, 1.00 equivalent) in THF-ds (0.250 mL), a solution of compound 14 (S-butyl thiobenzoate, 4.63 µL, 25.0 µmol, 1.00 equivalent) in THF-d₈ (0.250 mL) was added. The reaction mixture was heated at 30°C for 3 hours and ¹H NMR of the reaction mixture was directly analyzed. The yield of compound 15 was obtained based on -SCH₂-integral ratio of S-butyl thiobenzoate and CuSⁿ butyl. As a result, when the ratio of [Ph₂Cu][Mg₂Br₃(thf)₆] is 0.500 equivalents, the yield was 50%, whereas, when the ratio of [Ph₂Cu][Mg₂Br₃(thf)₆] is 1.00 equivalent, the yield was virtually 100% (quant.).

### Scheme (b)

To a J-young tube, a suspension of 0.5 M CuPh in THF (50.0 µL, 25.0 µmol, 1.00 equivalent) was added and THF was removed *in vacuo.* THF-d₈ (0.250 mL) was added, and thereafter, a solution of compound 14 (S-butyl thiobenzoate, 4.63 µL, 25.0 µmol, 1.00 equivalent) in THF-ds (0.250 mL) was added. The reaction mixture was heated at 30°C for 3 hours and ¹H NMR of the reaction mixture was directly analyzed. The yield of compound 15 was obtained based on -SCH₂-integral ratio of S-butyl thiobenzoate and CuSⁿ butyl. As a result, the yield was a trace amount.

### Scheme (c)

To a J-young tube, a suspension of 0.5 M CuPh in THF (25.0 µL, 12.5 µmol, 0.500 equivalents) was added and THF was removed *in vacuo.* A solution of [Ph₂Cu][Mg₂Br₃(thf)₆] (11.7 mg, 12.5 µmol, 0.500 equivalents) in THF-ds (0.250 mL) was added, and thereafter, a solution of compound 14 (S-butyl thiobenzoate, 4.63 µL, 25.0 µmol, 1.00 equivalent) in THF-d₈ (0.250 mL) was added. The reaction mixture was heated at 30°C for 3 hours and ¹H NMR of the reaction mixture was directly analyzed. The yield of compound 15 was obtained based on -SCH₂-integral ratio of S-butyl thiobenzoate and CuSⁿ butyl. As a result, the yield was 90%.

### Scheme (d)

To a J-young tube, a suspension of 0.5 M CuPh in THF (25.0 µL, 12.5 µmol, 0.500 equivalents) was added and THF was removed *in vacuo.* A solution of [Ph₂Cu][Mg₂Br₃(thf)₆] (11.7 mg, 12.5 µmol, 0.500 equivalents) in THF-ds (0.250 mL) was added, and thereafter, a solution of compound 16 (ethyl benzoate, 3.58 µL, 25.0 µmol, 1.00 equivalent) in THF-d₈ (0.250 mL) was added. The reaction mixture was heated at 30°C for 3 hours and ¹H NMR of the reaction mixture was directly analyzed. Compound 15 was not detected.

### Scheme (e)

To a solution of [Ph₂Cu][Mg₂Br₃(thf)₆] (70.4 mg, 0.0750 mmol, 30.0 mol%) in THF (3.15 mL), a suspension of 0.5 M CuPh in THF (0.350 mL, 0.175 mmol, 0.700 equivalents) was added, and subsequently, a solution of compound 17 (94.6 mg, 0.250 mmol, 1.00 equivalent) in THF (1.50 mL) was added. After the reaction mixture was heated at 30°C for 3 hours, a 1 M aqueous HCl solution (1 mL) was added to the reaction mixture to quench the reaction. A metal complex was removed by filtration through silica gel pad eluted with ethyl acetate. The yield of compound 18 was obtained by gas chromatography using triphenylethane (Ph₃CH) as the internal standard substance. As a result, the yield was 84%.

## Claims

1. A method for producing a ketone derivative (I) represented by the following formula (I): 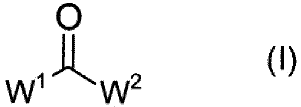
wherein W¹ and W² each independently represent an alkyl group that may have a substituent, an alkenyl group that may have a substituent, a cycloalkyl group that may have a substituent, a heterocycloalkyl group that may have a substituent, an aryl group that may have a substituent, a heteroaryl group that may have a substituent, an arylalkyl group that may have a substituent or an arylalkenyl group that may have a substituent,
the method comprising a step of mixing:
a thioester derivative (II) represented by the following formula (II): 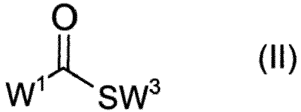
wherein W¹ is the same as defined above, and W³ represents an alkyl group that may have a substituent, an alkenyl group that may have a substituent, a cycloalkyl group that may have a substituent, a heterocycloalkyl group that may have a substituent, an aryl group that may have a substituent, a heteroaryl group that may have a substituent, an arylalkyl group that may have a substituent or an arylalkenyl group that may have a substituent;
a Grignard reagent (III) selected from the group consisting of
a Grignard reagent (IIIa) represented by the following formula (IIIa):
W²MgX (Illa)
wherein W² is the same as defined above, and X represents a halogen atom, and
a Grignard reagent (IIIb) represented by the following formula (IIIb):
W²MgX·LiCl (IIIb)
wherein W² and X are the same as defined above; and
a copper salt, to form the ketone derivative (I).

2. The method according to claim 1, wherein, in the step, the Grignard reagent (III) and the copper salt are mixed to form an organic copper reagent, and thereafter the thioester derivative (II) is mixed to contact the organic copper reagent and the thioester derivative (II) with each other.

3. The method according to claim 1 or 2, wherein an amount of the copper salt used is 0.1 mol or more and 1 mol or less relative to 1 mol of the Grignard reagent (III).

4. The method according to any one of claims 1 to 3, wherein the thioester derivative (II), the Grignard reagent (III) and the copper salt are mixed in a temperature range of 20°C or more and 60°C or less.

5. The method according to any one of claims 1 to 4,
wherein W² is an aryl group in which a carbon atom adjacent to each of two sides of a carbon atom having a bond of the aryl group has no substituent and the remaining carbon atoms may have a substituent; or a heteroaryl group in which a carbon atom or heteroatom adjacent to each of two sides of a carbon atom having a bond of the heteroaryl group has no substituent and the remaining carbon atoms or heteroatom(s) may have a substituent,
wherein, in the step, the thioester derivative (II), the Grignard reagent (III), the copper salt and a Grignard reagent (IV) represented by the following formula (IV):
W⁴MgX¹ (IV)
wherein W⁴ represents a phenyl group that has a substituent(s) at at least one of ortho positions and that may have a substituent(s) at a meta position(s) and/or a para position, and X¹ represents a halogen atom,
are mixed.

6. The method according to claim 5, wherein an amount of the Grignard reagent (IV) used is 0.01 mol or more and 1 mol or less relative to 1 mol of the Grignard reagent (III).

7. The method according to claim 5 or 6, wherein, in the step, the Grignard reagent (III) and the copper salt are mixed, and thereafter the Grignard reagent (IV) is mixed to form an organic copper reagent, and thereafter the thioester derivative (II) is mixed to contact the organic copper reagent and the thioester derivative (II) with each other.
